# EUROPEAN PATENT APPLICATION

(11) **EP 4 474 380 A1**
(43) Date of publication of application: **11.12.2024**
(21) Application number: 23769577.0
(22) Date of filing: 02.03.2023
(51) Int. Cl.: C07D 307/68, C07D 413/12, C07D 413/14, C08J 5/18, G02F 1/03

(54) **CHROMOPHORE AND METHOD FOR PREPARING SAME, ORGANIC ELECTRO-OPTIC MATERIAL, AND USE THEREOF**

(30) Priority: 14.03.2022 CN 202210248613
(71) Applicant: Huawei Technologies Co., Ltd., Shenzhen, Guangdong 518129 (CN)
(72) Inventor: LUO, Jingdong, Hong Kong 999077 (HK); ZHANG, Di, Hong Kong 999077 (HK); YAO, Zhanshi, Shenzhen, Guangdong 518129 (CN)
(74) Representative: Maiwald GmbH
(86) International application number: PCT/CN2023/079336
(87) International publication number: WO 2023/174071

(57) **Abstract**

This application relates to the field of electro-optic materials, and provides a chromophore and a method for preparing the chromophore, and an organic electro-optic material and application of the organic electro-optic material. The chromophore includes a conjugated bridge, and an acceptor and a donor respectively connected to two ends of the conjugated bridge via a chemical bond. The acceptor is 2-dicyanomethylidene-3-cyano-4-methyl-5,5-bis(4-fluorophenyl)-2,5-dihydrofuran. The chromophore provided in this application has a high β value and good chemical and thermal stability.

## Description

This application claims priority to Chinese Patent Application No. 202210248613.1, filed with the China National Intellectual Property Administration on March 14, 2022 and entitled "CHROMOPHORE AND METHOD FOR PREPARING CHROMOPHORE, AND ORGANIC ELECTRO-OPTIC MATERIAL AND APPLICATION OF ORGANIC ELECTRO-OPTIC MATERIAL", which is incorporated herein by reference in its entirety.

### TECHNICAL FIELD

This application relates to the field of electro-optic material technologies, and in particular, to a chromophore and a method for preparing the chromophore, and an organic electro-optic material and application of the organic electro-optic material.

### BACKGROUND

Electro-optic materials (Electro-optic materials) are optical functional materials with electro-optic effect. Currently, a most commonly used electro-optic material in the industry is a lithium niobate crystal. Development of the electro-optic material is mature, but the material has a low electro-optic coefficient (approximately 30 pm/V) and a high dielectric constant, which limits modulation efficiency and bandwidth of an electro-optic terminal device. This is also one of main limitations on development of optical communication technologies. In addition, processing of lithium niobate is difficult, a special etching process is needed, and it is difficult to directly grow a high-quality crystal on a silicon wafer, resulting in high preparation costs of the electro-optic terminal device. An organic electro-optic material has advantages of a high second-order nonlinear coefficient, a low dielectric constant, solution processability, easy hybrid integration and production with a silicon-based photonic integration platform, low processing costs, and the like. Compared with the lithium niobate crystal, the organic electro-optic material has advantages in terms of electro-optic coefficient, dielectric constant, processing simplicity, process compatibility, costs, and the like. Therefore, researching and developing an organic electro-optic material system with application prospects is one of urgent tasks in current development of the optical communication technologies, and can provide a new information processing and transmission technology with high bandwidth and low energy consumption, to serve a future data center, a telecommunication network, and the like.

The organic electro-optic material is usually formed by doping or chemically bonding a chromophore (chromophore) dipolar molecule with a high hyperpolarizability (β value) to a polymer. The chromophore is usually a conjugated system with an electron donor (donor) and an electron acceptor (acceptor) respectively connected to two ends, and the donor and the acceptor are connected using a conjugated bridge structure. To enable the mixed polymer to have an electro-optic modulation function, polarization needs to be performed on a mixed polymer: A temperature of the mixed polymer is raised to be around a glass transition temperature (Tg), and a strong direct current electric field is applied, so that the chromophore in the polymer has a specific orientation on a macroscopic scale; then, the temperature is lowered to a room temperature with the electric field maintained, so that the orientation of the chromophore is retained. After the polarization, the organic electro-optic material presents electro-optic effect on the macroscopic scale, and a refractive index of the organic electro-optic material is changed under effect of the external applied electric field.

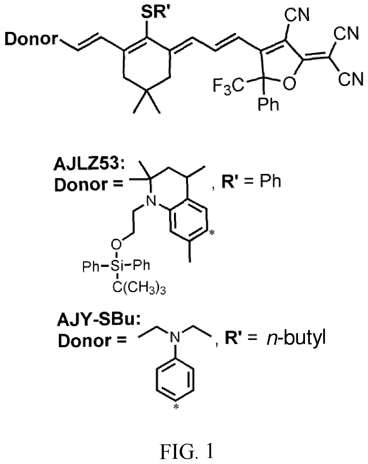

Currently, a chromophore dipolar molecule with a high β value in the industry is usually based on a trifluoromethyl (-CF3) substituted TCF acceptor, namely an acceptor CF3-TCF, that has a strong electron absorption capability. FIG. 1 shows two typical push-pull tetraene (push-pull tetraene) chromophores (AJLZ53 and AJY-SBu) that is based on a strong acceptor CF3-TCF. The two chromophores are currently optimal push-pull tetraene chromophores. A preparation procedure of this type of chromophore is usually based on step-by-step synthesis, and a synthesis procedure is as follows: First, from a donor end, a donor is modified and protected, and reacts with a modified conjugated bridge, then double bonds are added to extend a conjugated length of a main chain, and finally condensation with the strong acceptor (CF3-TCF) is performed. The push-pull tetraene chromophore based on the strong acceptor CF3-TCF has a high β value, which helps implement an electro-optic polymer with a high electro-optic coefficient. However, there are also some problems with the push-pull tetraene chromophore based on CF3-TCF. Because a product has a plurality of active sites and is likely to be attacked by impurities, chemical stability and thermal stability of the product (the chromophore based on the acceptor CF3-TCF and an electro-optic polymer including the chromophore) are limited. For example, under an alkaline condition, a dipolar molecule is very likely to decompose and deteriorate, and lose electro-optic properties. A thermal decomposition temperature of this type of chromophore is not high, resulting in a problem of thermal stability of the electro-optic polymer.

Therefore, how to efficiently obtain a chromophore dipolar molecule with a high β value through synthesis, design a highly stable electro-optic material system, and obtain a polarized polymer thin film with a high electro-optic coefficient through electric field polarization is an issue to be urgently resolved in the industry.

### SUMMARY

This application aims to provide a chromophore and a method for preparing the chromophore, and an organic electro-optic material including the chromophore and application of the organic electro-optic material, to resolve a problem of difficulty in achieving both a high β value and good chemical and thermal stability for an existing chromophore.

To achieve the objective of this application, the following technical solutions are used in this application.

A first aspect of this application provides a chromophore, including a conjugated bridge, and an acceptor and a donor respectively connected to two ends of the conjugated bridge via a chemical bond. The acceptor is 2-dicyanomethylidene-3-cyano-4-methyl-5,5-bis(4-fluorophenyl)-2,5-dihydrofuran (FP-TCF).

In the chromophore provided in this application, FP-TCF is used as an acceptor. FP-TCF has a strong electron accepting capability, which helps increase a β value of the chromophore. In addition, FP-TCF has weak reactivity with an alkali and other reagents, and a thermal decomposition temperature is high, so that chemical and thermal stability of the chromophore are improved.

In a possible implementation, the donor is a Michler's base derivative donor or a benzo [cd]indoline donor.

A structure of the Michler's base derivative donor is shown in Formula 1A, where R₁, R₂, R₃, and R₄ are separately selected from alkyl with 1 to 8 carbon atoms.

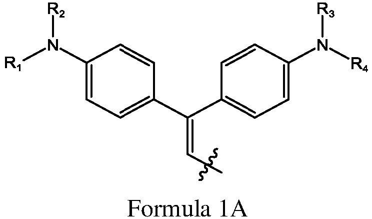

A structure of the benzo[cd]indoline donor is shown in Formula 2A, where R₅ is selected from alkyl with 1 to 8 carbon atoms.

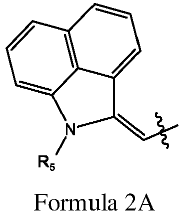

The Michler's base derivative donor shown in Formula 1A and the benzo[cd]indoline donor shown in Formula 2A have strong electron donating capabilities, and can effectively increase the β value of the chromophore in synergy with FP-TCF. Compared with the benzo[cd]indoline donor shown in Formula 2A, the Michler's base derivative donor shown in Formula 1A has more significant effect in increasing the β value of the chromophore.

In a possible implementation, the conjugated bridge is a first conjugated group including a 4,5-diphenyl-oxazol-2-ylsulfanyl group (4,5-diphenyl-oxazol-2-ylsulfanyl group) or a second conjugated group including a halogen atom. The 4,5-diphenyl-oxazol-2-ylsulfanyl group can form a large conjugated system, but with less π-π stacking. The halogen atom can provide a free electron. The two conjugated groups have good electron transfer capabilities, which facilitates transfer of an electron provided by the donor to the acceptor FP-TCF. 4,5-diphenyl-oxazol-2-ylsulfanyl has large steric hindrance, and can effectively reduce aggregation of chromophores and reduce π-π stacking of chromophore molecules after being connected to a conjugated group (a part other than 4,5-diphenyl-oxazol-2-ylsulfanyl in the conjugated bridge) that connects the acceptor and the donor. In addition, 4,5-diphenyl-oxazol-2-ylsulfanyl affects weak hydrogen bond interaction between molecules (for example, C-H···N or C-H···Cl), and increases a polar order parameter, so that the chromophore has a better orientation. Specifically, after a halogen atom is replaced by 4,5-diphenyl-oxazol-2-ylsulfanyl, bond length alternation (BLA) on a main chain is changed obviously. For example, according to single crystal data, BLA of a structure shown in Formula 5 is 0.04(9) Å, and BLA of a structure shown in Formula 6 obtained after sulfhydryl substitution is 0.029 Å. This indicates that the chromophore using the first conjugated group including the 4,5-diphenyl-oxazol-2-ylsulfanyl group as the conjugated bridge has a stronger cyanine-like resonance structure, and a finally obtained electro-optic material has better nonlinear optical properties and a higher electro-optic coefficient.

In a possible implementation, a structure of the first conjugated group is shown in Formula 3A:

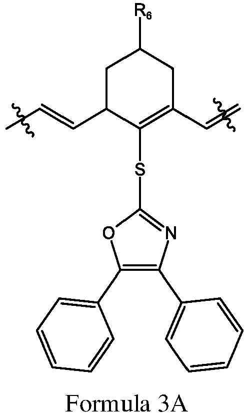

R₆ is selected from a hydrogen atom or alkyl with 1 to 8 carbon atoms.

A structure of the second conjugated group is shown in Formula 4A:

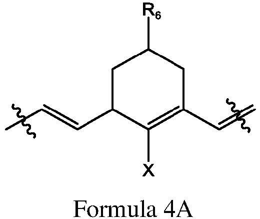

R₆ is selected from a hydrogen atom or alkyl with 1 to 8 carbon atoms, and X is a halogen atom.

Conjugated groups having structures shown in Formula 3A and Formula 4A include a plurality of consecutive double bonds. Double bonds on two sides are respectively connected to the acceptor and the donor of the chromophore, and an electron is transferred through the consecutive conjugated group.

In a possible implementation, the chromophore is selected from at least one of structures shown in Formula 5 to Formula 8:

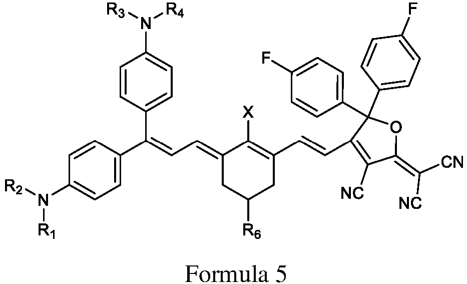

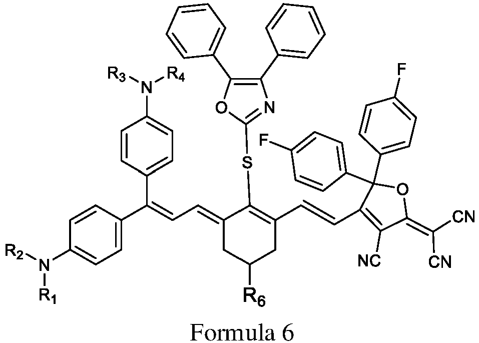

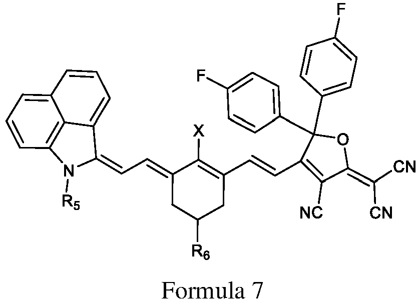

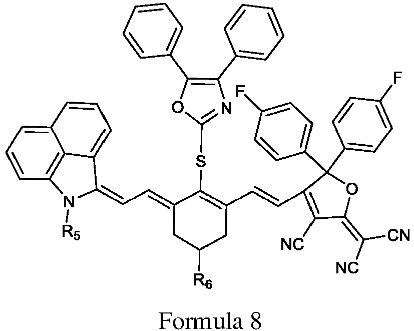

In the formulas, R₁, R₂, R₃, and R₄ are separately selected from alkyl with 1 to 8 carbon atoms, R₆ is selected from a hydrogen atom or alkyl with 1 to 8 carbon atoms, and X is a halogen atom.

Chromophores shown in Formula 5 to Formula 8 have ultrahigh β values. After the chromophores are doped or chemically bonded to polymers to form organic electro-optic materials, the ultrahigh β values help improve polarization effect of the organic electro-optic materials, so that the organic electro-optic materials present better photoelectric effect. In addition, the chromophores shown in the foregoing structures have excellent chemical and thermal stability.

In a possible implementation, R₁, R₂, R₃ and R₄ are the same and are selected from ethyl, n-propyl, n-butyl, n-pentyl, or n-hexyl, and R₆ is selected from an H atom, ethyl, isopropyl, or tert-butyl. R₁, R₂, R₃, and R₄ are the same, so that the donor has specific symmetry in structure, which facilitates synthesis of the chromophore and simplifies a synthesis procedure. R₁, R₂, R₃, and R₄ are selected from ethyl, n-propyl, n-butyl, n-pentyl, or n-hexyl. A chromophore framework includes a large quantity of aromatic groups and is a rigid framework, and an alkyl chain can improve solubility of the chromophore, so that the chromophore has solution processability. However, an excessively long molecular chain increases a molecular mass of the chromophore. To be specific, at a same mass percentage, a quantity concentration (a quantity of chromophores per unit volume) of the chromophore is decreased, and electro-optic properties are degraded; and when a quantity concentration is fixed, a doping mass is increased, and polarization difficulty and film forming quality are affected. In addition, compared with alkyl with a longer chain, R₁, R₂, R₃, and R₄ selected from these groups can reduce steric hindrance to some extent. R₆ is selected from an H atom, ethyl, isopropyl, or tert-butyl. Similarly, R₆ selected from alkyl helps improve solubility of the chromophore. Particularly, when R₆ is selected from tert-butyl, the chromophore has suitable solubility and a suitable molecular mass, so that solubility and photoelectric properties can be better balanced.

In a possible implementation, the chromophore is selected from one of the following structures:

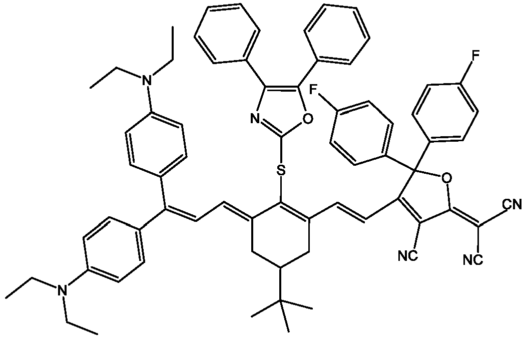

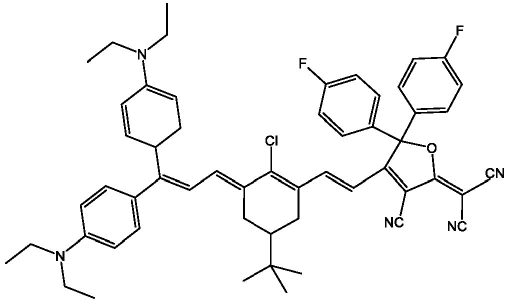

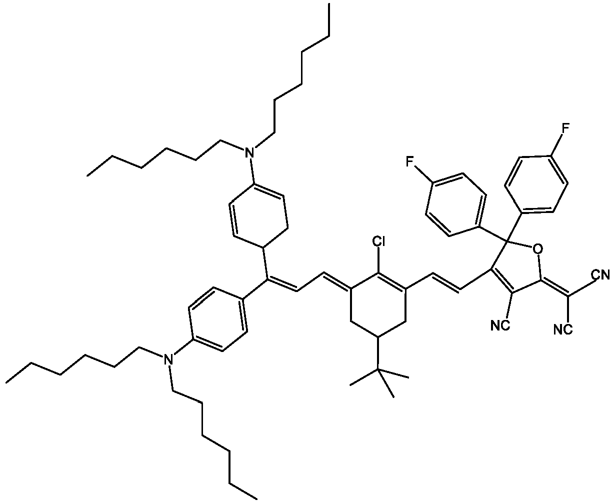

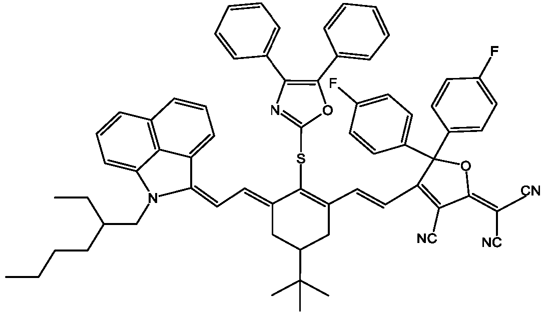

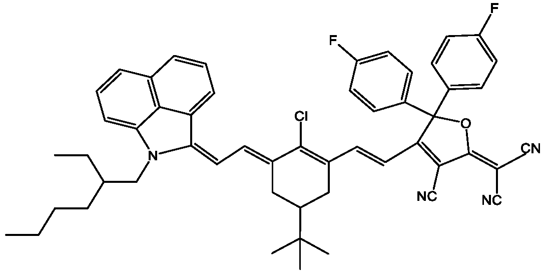

Chromophores shown in the foregoing structures have ultrahigh β values. Specifically, when a quantity concentration is 1.3×10²⁰ cm⁻³, at a wavelength of 1.3 µm, the β value of the chromophore reaches 8437×10⁻³⁰ esu. After the chromophores are doped or chemically bonded to polymers to form organic electro-optic materials, the ultrahigh β values help improve polarization effect of the organic electro-optic materials, so that the organic electro-optic materials present better photoelectric effect. In addition, the chromophores shown in the foregoing structures have weak reactivity with an alkali, thermal decomposition temperatures are higher than 200°C and may reach 281°C, and chemical and thermal stability are excellent.

A second aspect of this application provides a method for preparing a chromophore, including the following steps:
preparing a compound whose structure is shown in Formula 1B and a compound whose structure is shown in Formula 4B, where in Formula 1B, R₁, R₂, R₃, and R₄ are separately selected from alkyl with 1 to 8 carbon atoms, and in Formula 4B, R₆ is selected from a hydrogen atom or alkyl with 1 to 8 carbon atoms, and X is a halogen atom; 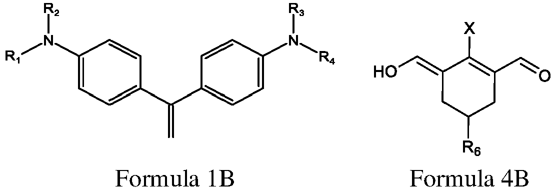
using a compound whose structure is shown in Formula 9 as a raw material and trimethylsilyl cyanide as a nucleophilic reagent to perform an addition reaction to obtain an intermediate 2,2-bis(4-fluorophenyl)-2-((trimethylsilyl)oxy)acetonitrile, adding methyl lithium to perform an addition reaction to obtain lithium (1,1-bis(4-fluorophenyl)-1-((trimethylsilyl)oxy)propan-2-ylidene)amide, and performing hydrolysis under an acid condition to obtain a compound whose structure is shown in Formula 10; and using the compound shown in Formula 10 as a raw material, adding malononitrile to perform an addition elimination reaction under catalysis of sodium ethoxide to obtain an imine intermediate, and adding malononitrile to perform a reaction to obtain a compound whose structure is shown in Formula 11; 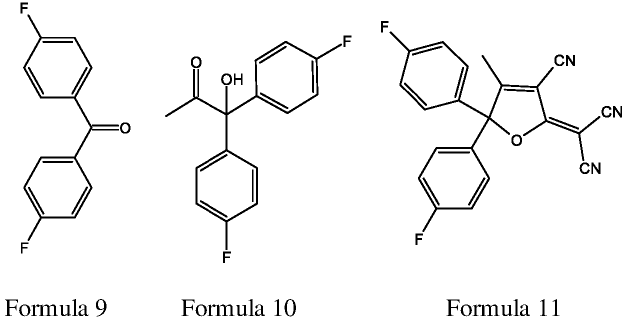
using the compound whose structure is shown in Formula 11 and the compound whose structure is shown in Formula 4B as raw materials to perform a condensation reaction to obtain a compound whose structure is shown in Formula 12; and 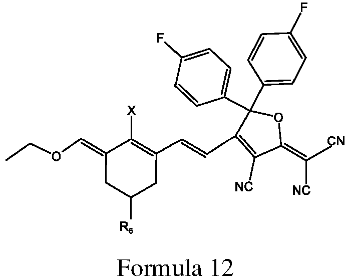
using the compound whose structure is shown in Formula 12 and the compound whose structure is shown in Formula 1B as raw materials to perform a condensation reaction to obtain a compound whose structure is shown in Formula 5; or using the compound whose structure is shown in Formula 12 and a compound whose structure is shown in Formula 2B as raw materials to perform a condensation reaction to obtain a compound whose structure is shown in Formula 7.

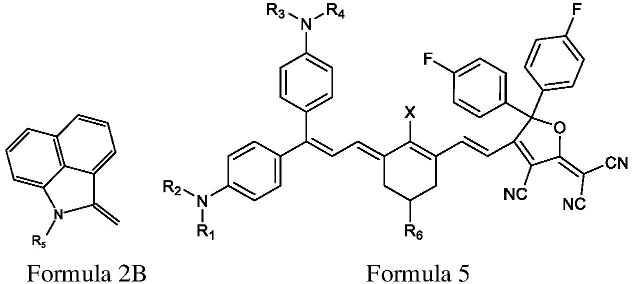

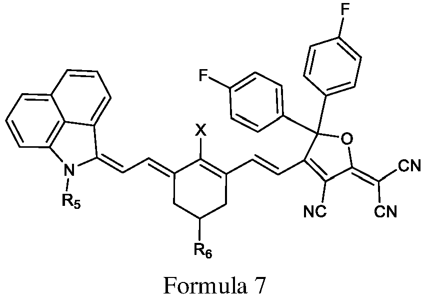

According to the method for preparing the chromophore provided in this application, two consecutive multiple-step one-pot reactions may be used to synthesize an acceptor 2-dicyanomethylidene-3-cyano-4-methyl-5,5-bis(4-fluorophenyl)-2,5-dihydrofuran (FP-TCF), and a total yield is 50%, which is far higher than that of an existing acceptor CF₃-TCF. On this basis, the acceptor is condensed with a conjugated bridge, and then an obtained compound is condensed with a donor to prepare the chromophore. There are few reaction steps and a high yield. In conclusion, the method for preparing the chromophore provided in this application has advantages of a simple process, a high yield, and low costs.

In a possible implementation, when an obtained compound is the compound shown in Formula 5, the method further includes: using the compound whose structure is shown in Formula 5 and a compound whose structure is shown in Formula 13 as raw materials to perform a nucleophilic substitution reaction to obtain a compound whose structure is shown in Formula 6.

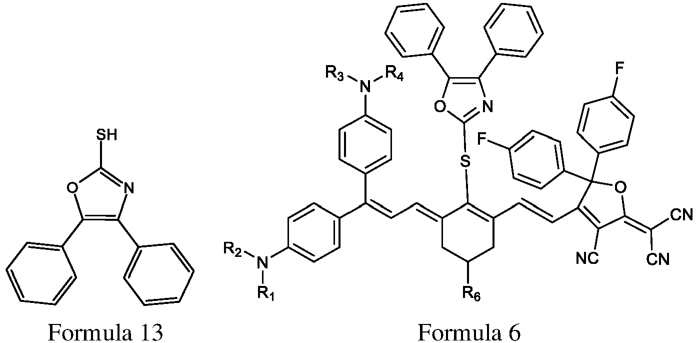

In this implementation, a halogen atom of the structure shown in Formula 5 is replaced by a 4,5-diphenyloxazol-2-ylsulfanyl group to obtain the structure shown in Formula 6. 4,5-diphenyl-oxazol-2-ylsulfanyl has large steric hindrance, and can effectively reduce aggregation of chromophores and reduce π-π stacking of chromophore molecules after being connected to a conjugated group (a part other than 4,5-diphenyl-oxazol-2-ylsulfanyl in the conjugated bridge) that connects the acceptor and the donor. In addition, 4,5-diphenyl-oxazol-2-ylsulfanyl affects weak hydrogen bond interaction between molecules (for example, C-H ... N or C-H ... CI), and increases a polar order parameter, so that the chromophore has a better orientation. Specifically, after the halogen atom is replaced by 4,5-diphenyl-oxazol-2-ylsulfanyl, bond length alternation (BLA) on a main chain is changed obviously. For example, according to single crystal data, BLA of the structure shown in Formula 5 is 0.04(9) Å, and BLA of the structure shown in Formula 6 obtained after sulfhydryl substitution is 0.029 Å. This indicates that the chromophore using a first conjugated group including the 4,5-diphenyl-oxazol-2-ylsulfanyl group as the conjugated bridge has a stronger cyanine-like resonance structure, and a finally obtained polymer material has better nonlinear optical properties and a higher electro-optic coefficient.

In a possible implementation, when an obtained compound is a compound shown in Formula 6, the method further includes: using the compound whose structure is shown in Formula 7 and a compound whose structure is shown in Formula 13 as raw materials to perform a nucleophilic substitution reaction to obtain a compound whose structure is shown in Formula 8.

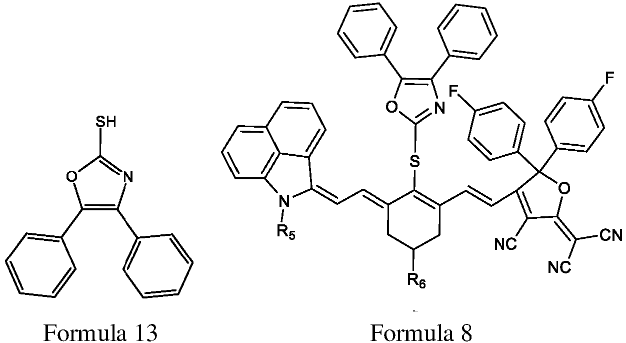

In this implementation, a halogen atom of the structure shown in Formula 7 is replaced by a 4,5-diphenyloxazol-2-ylsulfanyl group to obtain the structure shown in Formula 8, so that π-π stacking is reduced. In addition, slightly adjusted bond length alternation can improve nonlinear optical properties of an organic electro-optic material including the chromophore that is based on 2-dicyanomethylidene-3-cyano-4-methyl-5,5-bis(4-fluorophenyl)-2,5-dihydrofuran (FP-TCF), to have a higher electro-optic coefficient.

In a possible implementation, the compound whose structure is shown in Formula 1B is prepared by using the following method: making a compound shown in Formula 14 react with methyl lithium to obtain the compound whose structure is shown in Formula 1B. The reaction is shown in the following formula:

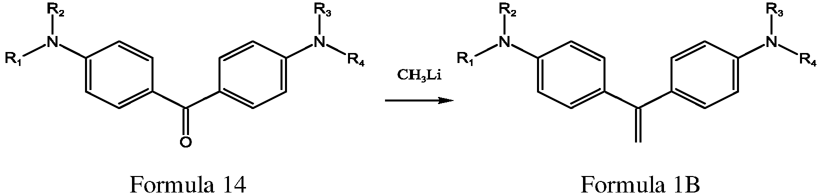

With the use of this method, the Michler's base derivative shown in Formula 1B can be prepared in one step, and a yield can reach 95%.

In a possible implementation, the compound whose structure is shown in Formula 4B is prepared by using the following method: making a compound shown in Formula 15 react with phosphoryl chloride or phosphoryl bromide to obtain the compound whose structure is shown in Formula 4B. The reaction is shown in the following formula:

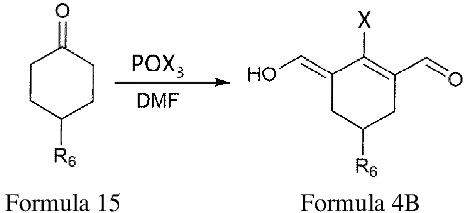

In Formula 4B and POX₃, X is a chlorine atom or a bromine atom.

A third aspect of this application provides an organic electro-optic material, including a polymer and a chromophore. The chromophore is doped to the polymer, or the chromophore is chemically bonded to the polymer. The chromophore is the chromophore according to the first aspect of this application or the chromophore obtained by using the method according to the second aspect of this application.

According to the organic electro-optic material provided in this application, the chromophore is the chromophore provided in the first aspect of this application or the chromophore obtained in the second aspect of this application. Because the chromophore has a high β value and excellent chemical and thermal stability, nonlinear optical properties of the organic electro-optic material can be improved, to have a higher electro-optic coefficient.

A fourth aspect of this application provides an electro-optic terminal device. At least one component of the electro-optic terminal device includes the organic electro-optic material according to the first aspect of this application.

The electro-optic terminal device provided in this application includes a component including the organic electro-optic material, so that the component can better present electro-optic properties, to improve use performance of the electro-optic terminal device.

In a possible implementation, the electro-optic terminal device includes an electro-optic modulator, an electric field sensor, or a wireless signal receiver. Such an electro-optic modulator has advantages of a low drive voltage, high bandwidth, and a small size.

### BRIEF DESCRIPTION OF DRAWINGS

FIG. 1 is a diagram of chemical structures of two typical push-pull tetraene (push-pull tetraene) chromophores (AJLZ53 and AJY-SBu) based on a strong acceptor CF₃-TCF in a conventional technology;
FIG. 2 is a diagram of a structure of an electro-optic modulator according to an embodiment of this application;
FIG. 3 is a flowchart of a preparation process of a chromophore according to an embodiment of this application;
FIG. 4 is a two-dimensional ¹H-¹H ROESY overall spectrum of a chromophore according to Embodiment 1 of this application;
FIG. 5 is a partial enlarged view of FIG. 4 of this application;
FIG. 6 is a diagram of states of an organic electro-optic material including a chromophore and a polymer before and after polarization according to an embodiment of this application;
FIG. 7 is a diagram of ultraviolet-visible-near infrared spectra of a chromophore (M1-FP-ON, corresponding to (a)) according to Embodiment 1 of this application and a push-pull tetraene chromophore (AJY-SBu, corresponding to (b)) based on CF₃-TCF; and
FIG. 8 is a thermogravimetric analysis (TGA) diagram of a chromophore based on FP-TCF according to an embodiment of this application.

### DESCRIPTION OF EMBODIMENTS

To make technical problems to be resolved, technical solutions, and beneficial effects in this application clearer, the following further describes this application in detail with reference to embodiments. It should be understood that the specific embodiments described herein are merely used to explain this application but are not intended to limit this application.

In this application, the term "and/or" describes an association relationship between associated objects and indicates that three relationships may exist. For example, A and/or B may indicate the following cases: Only A exists, both A and B exist, and only B exists. A and B may be singular or plural. The character "/" generally indicates an "or" relationship between associated objects.

In this application, "at least one" means one or more, and "a plurality of" means two or more. "At least one of the following items (pieces)" or a similar expression thereof means any combination of these items, including a single item (piece) or any combination of a plurality of items (pieces). For example, "at least one of a, b, or c" or "at least one of a, b, and c" may indicate: a, b, c, a-b (that is, a and b), a-c, b-c, or a-b-c, where a, b, and c may be singular or plural.

It should be understood that in various embodiments of this application, sequence numbers of the foregoing procedures do not mean execution sequences. Some or all of the steps may be performed in parallel or in sequence. The execution sequences of the procedures should be determined according to functions and internal logic of the procedures, and shall not be construed as any limitation on the implementation procedures of embodiments of this application.

Terms used in embodiments of this application are merely for the purpose of describing specific embodiments, and are not intended to limit this application. The terms "a", "said", and "the" of singular forms used in embodiments and the appended claims of this application are also intended to include plural forms, unless otherwise specified in the context clearly.

In this specification, "include", "comprise", "involve", "contain", "have", or another variant is intended to cover non-closed inclusion, and no distinction is made between these terms. The term "include" means that other steps and elements that do not affect a final result may be added. The term "include" further includes the terms "consist of" and "basically consist of". A combination and a method/process in this application include, consist of, and basically consist of necessary elements and limitation items described in this specification and any additional or optional parts, components, steps, or limitation items described in this specification.

The terms "first" and "second" are merely used for descriptive purposes, and are used to distinguish between purposes such as substances, but shall not be understood as an indication or implication of relative importance or an implicit indication of a quantity of indicated technical features. For example, without departing from the scope of embodiments of this application, a first XX may also be referred to as a second XX. Similarly, a second XX may also be referred to as a first XX. Therefore, a feature defined by "first" or "second" may explicitly or implicitly include one or more features.

The term "chromophore" is also referred to as colored molecule, and is "chromophore" in English.

The term "polymethines" is "polymethines" in English.

The term "heptamethine" is "heptamethine" in English.

The term "dipolar polyene" is "dipolar polyene" in English.

The term "β value" is an abbreviation for "hyperpolarizability", may also be expressed as "beta value", and indicates a hyperpolarizability.

The term "Tg" is an abbreviation for "glass transition temperature", and indicates a glass transition temperature.

The term "push-pull tetraene" is "push-pull tetraene" in English.

The term "TGA" is an abbreviation for "thermogravimetric analysis", and indicates thermogravimetric analysis.

The term "ROESY" is an abbreviation for "rotating frame overhauser enhancement spectroscopy", and indicates a rotating frame NOE spectrum.

The term "DMSO" is an abbreviation for "dimethyl sulfoxide", and indicates dimethyl sulfoxide.

The term "P(S-co-MMA)" is an abbreviation for "poly(styrene-co-methyl methacrylate)", and indicates a methyl methacrylate-styrene copolymer.

The term "MZ" is an abbreviation for "Mach-Zehnder", and indicates Mach-Zehnder.

The term "FP" indicates bis(4-fluorophenyl).

The term "TCF" is an abbreviation for "2-dicyanomethylidene-3-cyano-4,5,5-trimethyl-2,5-dihydrofuran", and indicates 2-dicyanomethylidene-3-cyano-4,5,5-trimethyl-2,5-dihydrofuran.

An organic electro-optic material has advantages of a high second-order nonlinear coefficient, a low dielectric constant, solution processability, easy hybrid integration and production with a silicon-based photonic integration platform, low processing costs, and the like, and is one of key photoelectric functional materials for designing and developing a next-generation high-speed electro-optic modulation, optical switching device, and the like with high bandwidth and a low drive voltage. The organic electro-optic material, also referred to as an electro-optic polymer, may be formed by doping or chemically bonding a chromophore with a high β value to a polymer. The organic electro-optic material has electro-optic effect after polarization, and may be used to implement an electro-optic terminal device that is based on the organic electro-optic material, and used in a system such as a future data center or telecommunication network.

In a possible implementation, the electro-optic terminal device may be an electro-optic modulator, an electric field sensor, or a wireless signal receiver. The organic electro-optic material formed by doping or chemically bonding the chromophore to the polymer is used to produce at least one component of the electro-optic terminal device. For example, a phase shifter of the electro-optic modulator is made of the organic electro-optic material formed by doping or chemically bonding the chromophore to the polymer, an electro-optic conversion area of the electric field sensor is made of the organic electro-optic material formed by doping or chemically bonding the chromophore to the polymer, or an electro-optic conversion area of the wireless signal receiver is made of the organic electro-optic material formed by doping or chemically bonding the chromophore to the polymer.

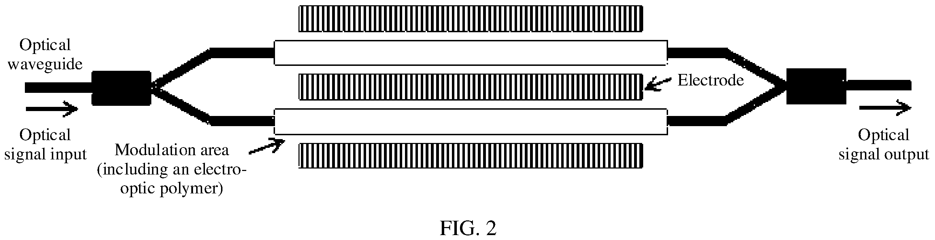

In an implementation, an electro-optic modulator is used as an example for description. Refer to FIG. 2. The electro-optic modulator has an MZ interferometer structure. An electro-optic polymer formed based on a chromophore is used in an MZ interferometer and forms at least one arm of the MZ interferometer, which is used to modulate an optical signal to form a modulation area of the electro-optic modulator. An effective refractive index of a waveguide in this area may be affected by an electric field applied by an electrode, and a phase of an optical signal of the arm may be changed. In this way, strength or a phase of an optical signal at an optical output end can be changed, to implement conversion from an electrical signal into an optical signal. In addition to the MZ interferometer structure, a microring resonant cavity structure, a Fabry-Perot (FP) resonant cavity, a photonic crystal, and other optical structures may also be used to implement an electro-optic modulation device that is based on an electro-optic polymer.

The following describes the organic electro-optic material in detail. The organic electro-optic material includes a polymer. As a main component of the organic electro-optic material, the polymer has electro-optic effect through polarization after a chromophore is doped or chemically bonded to the polymer, and may be used to obtain an electro-optic terminal device with high bandwidth, high efficiency, and low costs that is based on the organic electro-optic material. The polymer used in the organic electro-optic material is not specifically limited in embodiments of this application, and a currently known polymer in the field of organic electro-optic materials may be used. For example, the polymer is P(S-co-MMA).

The organic electro-optic material further includes a chromophore. As components of the organic electro-optic material, the chromophore and the polymer may be mixed in a form of physical doping, or may be combined in a chemical bonding manner, to form an organic compound system. The chromophore is usually a conjugated system with an electron donor (donor) and an electron acceptor (acceptor) respectively connected to two ends, and the donor and the acceptor are connected using a conjugated bridge structure. In other words, the chromophore includes a conjugated bridge, and an acceptor and a donor respectively connected to two ends of the conjugated bridge via a chemical bond. A β value, thermal stability, and chemical stability of the chromophore are important indicators for indicating properties of the chromophore. Currently, representatives of chromophores with good properties in the industry include push-pull tetraene (push-pull tetraene) chromophores AJLZ53 and AJY-SBu that are based on a strong acceptor CF₃-TCF. These two chromophores have high β values, and can provide good electro-optic properties for an organic electro-optic material. However, a preparation process of such a chromophore is complex, and stability, for example, chemical and thermal stability, is poor. In view of this, an embodiment of this application provides a chromophore molecule with a high β value, good chemical and thermal stability, and a high synthetic yield, used to synthesize an organic electro-optic material with a high electro-optic coefficient, high stability, and low costs, so as to implement an electro-optic terminal device with high bandwidth, high efficiency, and low costs that is based on the organic electro-optic material.

Specifically, the chromophore is a heptamethine chromophore, and 2-dicyanomethylidene-3-cyano-4-methyl-5,5-bis(4-fluorophenyl)-2,5-dihydrofuran (also referred to as FP-TCF below) is used as an acceptor, namely, an acceptor FP-TCF. A structure of FP-TCF is as follows:

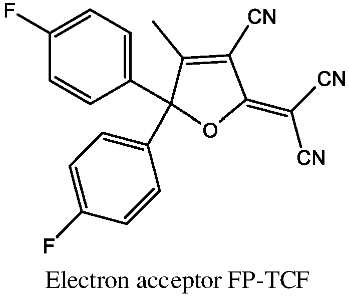

FP-TCF has a strong electron accepting capability, which helps increase the β value of the chromophore. In addition, FP-TCF has weak reactivity with an alkali and other reagents, and a thermal decomposition temperature is high, so that chemical and thermal stability of the chromophore are improved.

In a possible implementation, a donor of the chromophore provided in this embodiment of this application is a Michler's base derivative donor or a benzo [cd]indoline donor. Specifically, a structure of the Michler's base derivative donor is shown in Formula 1A. R₁, R₂, R₃, and R₄ are separately selected from alkyl with 1 to 8 carbon atoms.

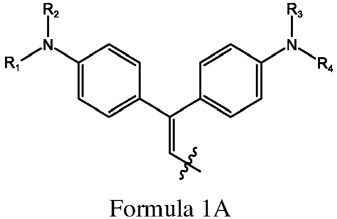

A structure of the benzo[cd]indoline donor is shown in Formula 2A. R₅ is selected from alkyl with 1 to 8 carbon atoms.

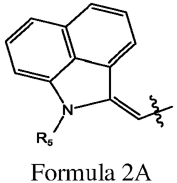

The Michler's base derivative (Michler's base derivative) donor shown in Formula 1A and the benzo[cd]indoline (benzo[cd]indoline) donor shown in Formula 2A have strong electron donating capabilities, and can effectively increase the β value of the chromophore in synergy with FP-TCF. Compared with the benzo[cd]indoline donor shown in Formula 2A, the Michler's base derivative donor shown in Formula 1A has more significant effect in increasing the β value of the chromophore. It should be understood that structures of the Michler's base derivative donor shown in Formula 1A and the benzo [cd]indoline donor shown in Formula 2A show binding sites on which the Michler's base derivative donor and the benzo[cd]indoline donor are bound to a conjugated bridge. Original structures of the Michler's base derivative donor shown in Formula 1A and the benzo[cd]indoline donor shown in Formula 2A are respectively shown in Formula 1B and Formula 2B:

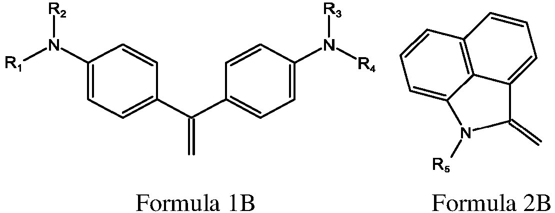

In Formula 1A and Formula 1B, R₁, R₂, R₃, and R₄ are separately selected from alkyl with 1 to 8 carbon atoms. Similarly, in Formula 2A and Formula 2B, R₅ is selected from alkyl with 1 to 8 carbon atoms. The chromophore includes a large quantity of aromatic groups, and these aromatic groups form a rigid framework. Introducing an alkyl group can improve solubility of the chromophore, so that the chromophore has solution processability. However, an excessively long molecular chain increases a molecular mass of the chromophore. To be specific, at a same mass percentage, a quantity concentration (a quantity of chromophores per unit volume) of the chromophore is decreased, and electro-optic properties are degraded; and when a quantity concentration is fixed, a doping mass is increased, and polarization difficulty and film forming quality are affected. Therefore, in this embodiment of this application, R₁, R₂, R₃, and R₄ in Formula 1A and Formula 1B, and R₅ in Formula 2A and Formula 2B are selected from alkyl with 1 to 8 carbon atoms.

In a possible implementation, in structures shown in Formula 1A and Formula 1B, R₁, R₂, R₃, and R₄ are the same, so that the structure of the Michler's base derivative donor shown in Formula 1B has specific symmetry, which helps simplify a synthetic process of the chromophore.

In a possible implementation, R₁, R₂, R₃, and R₄ are selected from ethyl, n-propyl, n-butyl, n-pentyl, or n-hexyl. These alkyl chains can improve solubility of the chromophore, so that the chromophore has solution processability, and electro-optic properties, polarization difficulty, and film forming quality of the chromophore are considered. In addition, compared with alkyl with a longer chain, R₁, R₂, R₃, and R₄ selected from these groups can reduce steric hindrance to some extent. For example, the Michler's base derivative shown in Formula 1B may be selected from the following structures:

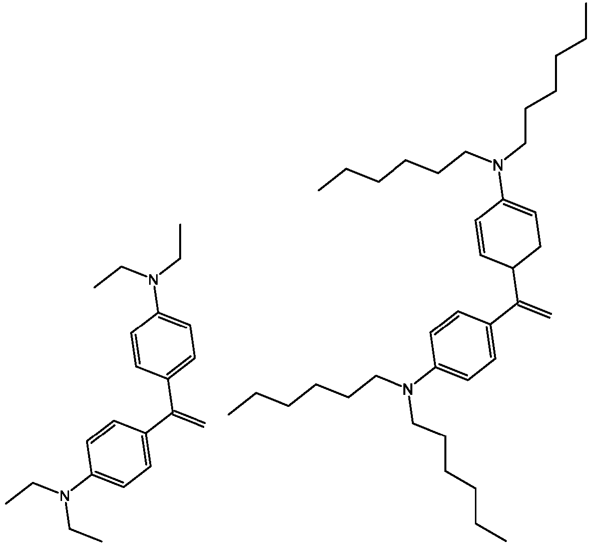

In a possible implementation, in the structure shown in Formula 2A or Formula 2B, R₅ is selected from ethyl, n-propyl, n-butyl, n-pentyl, n-hexyl, or 2-ethylhexyl, to ensure good solubility of the chromophore, and to consider electro-optic properties, polarization difficulty, and film forming quality of the chromophore. For example, the benzo[cd]indoline shown in Formula 2B may be selected from the following structure:

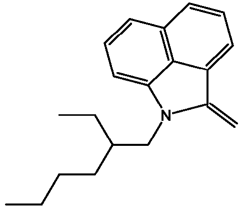

In this embodiment of this application, the acceptor and the donor of the chromophore are bonded by a conjugated bridge. In a possible implementation, the conjugated bridge is a first conjugated group including a 4,5-diphenyl-oxazol-2-ylsulfanyl group (4,5-diphenyl-oxazol-2-ylsulfanyl group). The 4,5-diphenyl-oxazol-2-ylsulfanyl group can increase a volume of the first conjugated group, but with less π-π stacking, which facilitates transfer of an electron provided by the donor to the acceptor FP-TCF.

In some embodiments, a structure of the first conjugated group is shown in Formula 3A:

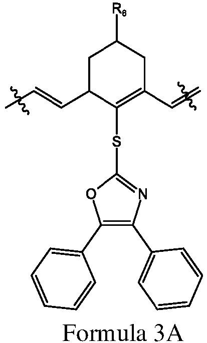

R₆ is selected from a hydrogen atom or alkyl with 1 to 8 carbon atoms.

The foregoing conjugated structure includes the 4,5-diphenyl-oxazol-2-ylsulfanyl group. 4,5-diphenyloxazol-2-ylsulfanyl has large steric hindrance, and can effectively reduce aggregation of chromophores and reduce *π-*π stacking of chromophore molecules after being connected to a conjugated group (a part other than 4,5-diphenyloxazol-2-ylsulfanyl in the conjugated bridge) that connects the acceptor and the donor. In addition, 4,5-diphenyloxazol-2-ylsulfanyl affects weak hydrogen bond interaction between molecules (for example, C-H···N or C-H···Cl), and increases a polar order parameter, so that the chromophore has a better orientation. Specifically, after a halogen atom is replaced by 4,5-diphenyl-oxazol-2-ylsulfanyl, bond length alternation (BLA) on a main chain is changed obviously. For example, according to single crystal data, BLA of a structure shown in Formula 5 is 0.04(9) Å, and BLA of a structure shown in Formula 6 obtained after sulfhydryl substitution is 0.029 Å. This indicates that the chromophore using the first conjugated group including the 4,5-diphenyl-oxazol-2-ylsulfanyl group as the conjugated bridge has a stronger cyanine-like resonance structure, and a finally obtained electro-optic material has better nonlinear optical properties and a higher electro-optic coefficient.

It should be understood that the structure of the first conjugated group shown in Formula 3A shows two binding sites on which the first conjugated group is bound to the acceptor and the donor.

In a possible implementation, R₆ is selected from alkyl, which helps improve solubility of the chromophore. For example, when R₆ is selected from tert-butyl, the chromophore has suitable solubility and a suitable molecular mass, so that solubility and photoelectric properties can be better balanced.

In another possible implementation, the conjugated bridge is a second conjugated group including a halogen atom. The halogen atom can provide a free electron, to facilitate transfer of an electron provided by the donor to the acceptor FP-TCF.

In some embodiments, a structure of the second conjugated group is shown in Formula 4A:

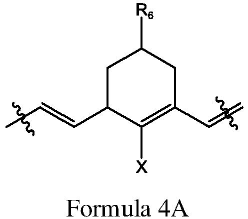

R₆ is selected from a hydrogen atom or alkyl with 1 to 8 carbon atoms, and X is a halogen atom.

The foregoing conjugated structure includes a halogen atom, and the halogen atom has a specific electron absorbing capability. This affects a push-pull structure of a main chain. In addition, the halogen atom is an active group and may further react. Halogen atoms are replaced by different sulfhydryl groups, to modify the chromophore. For example, X in Formula 4A is replaced by a 4,5-diphenyl-oxazol-2-ylsulfanyl group. To be specific, Formula 6 is obtained after Formula 5 is modified by using 4,5-diphenyl-oxazol-2-ylsulfanyl, so that both solubility and electro-optic properties are improved. It should be understood that the structure of the first conjugated group shown in Formula 4A shows two binding sites on which the first conjugated group is bound to the acceptor and the donor. An original structure of the first conjugated group shown in Formula 4A is shown in Formula 4B:

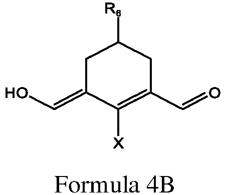

R₆ is selected from a hydrogen atom or alkyl with 1 to 8 carbon atoms, and X is a halogen atom.

In a possible implementation, the halogen atom may be a chlorine atom or a bromine atom. For example, X is a chlorine atom. In this case, preparation and processing are facilitated. This is because dialdehyde is obtained through a Vilsmeier-Haack (Vilsmeier-Haack) reaction, and phosphoryl chloride is usually used for the reaction. Phosphoryl chloride is liquid and phosphoryl bromide is solid. Liquid is easy to operate because materials are added step-by-step during experiment. In addition, compared with the bromine atom, the chlorine atom has a lower molecular mass, and for a halogen-containing chromophore, a doping mass percentage is low. Therefore, the chlorine atom is preferred for X in this application.

In a possible implementation, R₆ is selected from a hydrogen atom or tert-butyl. When R₆ is selected from tert-butyl, solubility of the chromophore can be effectively improved. For example, a compound corresponding to the second conjugated group in Formula 4B may be selected from the following structures:

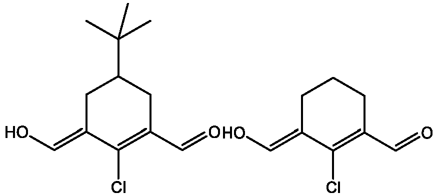

The conjugated group having the structure shown in Formula 4B includes a plurality of consecutive double bonds. Double bonds on two sides are respectively connected to the acceptor and the donor of the chromophore, and an electron is transferred through the consecutive conjugated group. On this basis, a 4,5-diphenyl-oxazol-2-ylsulfanyl group or a halogen atom is introduced to further improve an electron transfer capability.

In a possible implementation, the chromophore is selected from at least one of structures shown in Formula 5 to Formula 8:

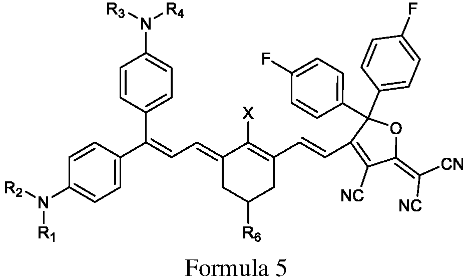

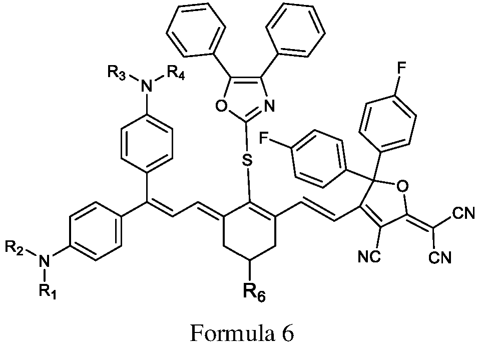

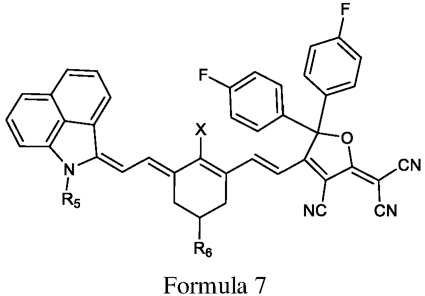

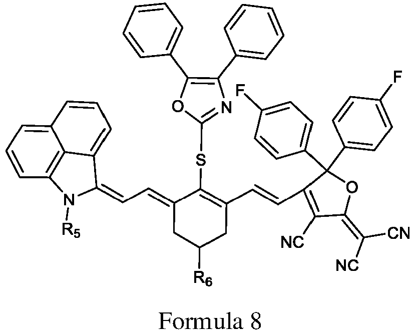

In the formulas, R₁, R₂, R₃, and R₄ are separately selected from alkyl with 1 to 8 carbon atoms, R₆ is selected from a hydrogen atom or alkyl with 1 to 8 carbon atoms, and X is a halogen atom.

Chromophores shown in Formula 5 to Formula 8 have ultrahigh β values. After the chromophores are doped or chemically bonded to polymers to form organic electro-optic materials, the ultrahigh β values help improve polarization effect of the organic electro-optic materials, so that the organic electro-optic materials present better photoelectric effect. In addition, the chromophores shown in the foregoing structures have excellent chemical and thermal stability.

In a possible implementation, in Formula 5 and Formula 6, R₁, R₂, R₃, and R₄ are the same and are selected from ethyl, n-propyl, n-butyl, n-pentyl, or n-hexyl. R₁, R₂, R₃, and R₄ are the same, so that the donor has specific symmetry in structure, which facilitates synthesis of the chromophore and simplifies a synthesis procedure. For example, R₁, R₂, R₃, and R₄ are selected from ethyl, n-propyl, n-butyl, n-pentyl, or n-hexyl, so that the chromophore has suitable solubility and a suitable molecular mass. A chromophore framework includes a large quantity of aromatic groups and is a rigid framework, and an alkyl chain can improve solubility of the chromophore, so that the chromophore has solution processability. However, an excessively long molecular chain increases a molecular mass of the chromophore. To be specific, at a same mass percentage, a quantity concentration (a quantity of chromophores per unit volume) of the chromophore is decreased, and electro-optic properties are degraded; and when a quantity concentration is fixed, a doping mass is increased, and polarization difficulty and film forming quality are affected. In addition, compared with alkyl with a longer chain, R₁, R₂, R₃, and R₄ selected from these groups can reduce steric hindrance to some extent.

In Formula 7 and Formula 8, R₅ is selected from a longer alkyl chain such as 2-ethylhexyl to improve solubility of the chromophore.

In Formula 5 to Formula 8, R₆ is selected from tert-butyl to improve solubility of the chromophore. In Formula 5 and Formula 7, X is selected from a chlorine atom to facilitate preparation and processing. This is because dialdehyde is obtained through a Vilsmeier-Haack (Vilsmeier-Haack) reaction, and phosphoryl chloride is usually used for the reaction. Phosphoryl chloride is liquid and phosphoryl bromide is solid. Liquid is easy to operate because materials are added step-by-step during experiment. In addition, compared with a bromine atom, the chlorine atom has a lower molecular mass, and for a halogen-containing chromophore, a doping mass percentage is low. Therefore, the chlorine atom is preferred for X in this application.

In a possible implementation, the chromophore is selected from one of the following structures:

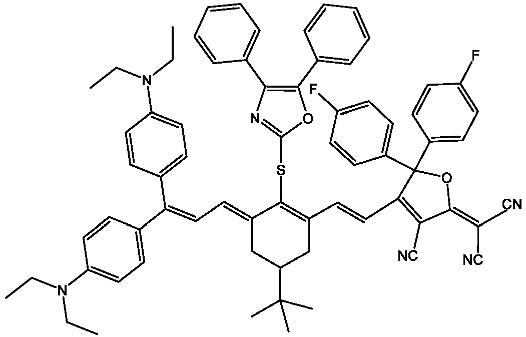

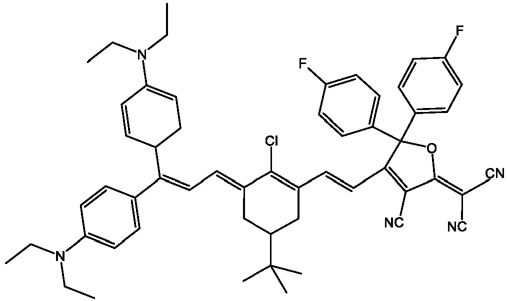

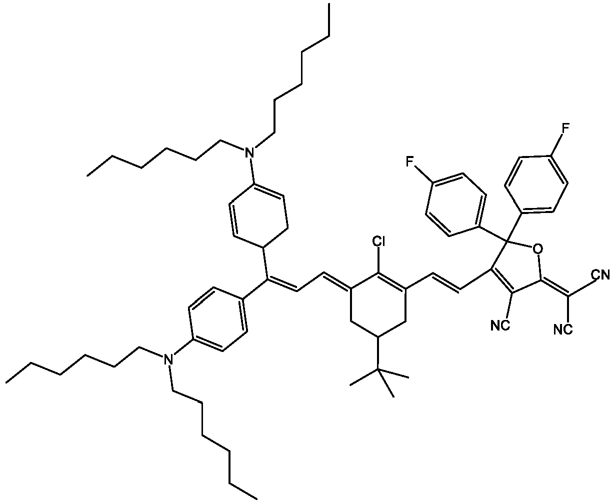

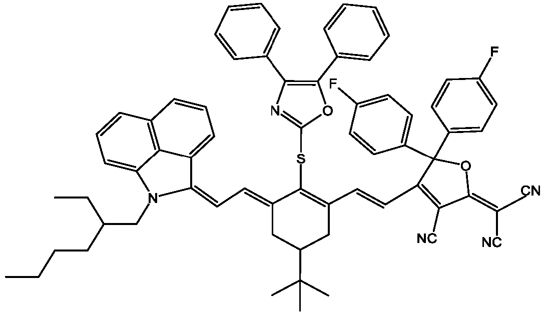

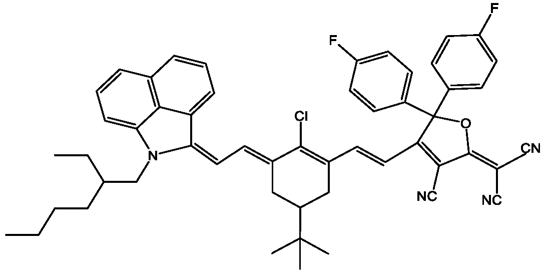

Chromophores shown in the foregoing structures have ultrahigh β values. Specifically, when a quantity concentration is 1.3×10²⁰ cm⁻³, at a wavelength of 1.3 µm, the β value of the chromophore reaches 8437×10⁻³⁰ esu. After the chromophores are doped or chemically bonded to polymers to form organic electro-optic materials, the ultrahigh β values help improve polarization effect of the organic electro-optic materials, so that the organic electro-optic materials present better photoelectric effect. In addition, the chromophores shown in the foregoing structures have weak reactivity with an alkali, thermal decomposition temperatures are higher than 200°C and may reach 281°C, and chemical and thermal stability are excellent.

A push-pull tetraene chromophore based on CF₃-TCF has poor stability and is difficult to synthesize. A donor and a conjugated bridge need to be modified before a reaction, a procedure is lengthy, and usually more than 10 steps of reaction are needed to obtain a final product, consuming a lot of time. A palladium precious metal catalyst is needed for some steps, causing high synthesis costs. In addition, it is difficult to purify intermediate products and final products, and a yield is low. It is difficult to satisfy basic requirements of continuous basic research, large-scale material processing and optimization, and device preparation. In view of this, an embodiment of this application further provides a method with a relatively simple process, to prepare the foregoing chromophore provided in embodiments of this application. According to the method for preparing the chromophore provided in this embodiment of this application, a consecutive synthesis method may be used, and there are few reaction steps, a short period, a high yield, a low operating condition requirement, and good reaction repeatability, to facilitate large-scale application. In addition, no precious metal catalyst is needed in a synthesis procedure of the chromophore, which helps reduce production costs. In view of this, an embodiment of this application provides a method for preparing a chromophore that is based on the foregoing FP-TCF, to obtain a synthesis method with a simplified procedure.

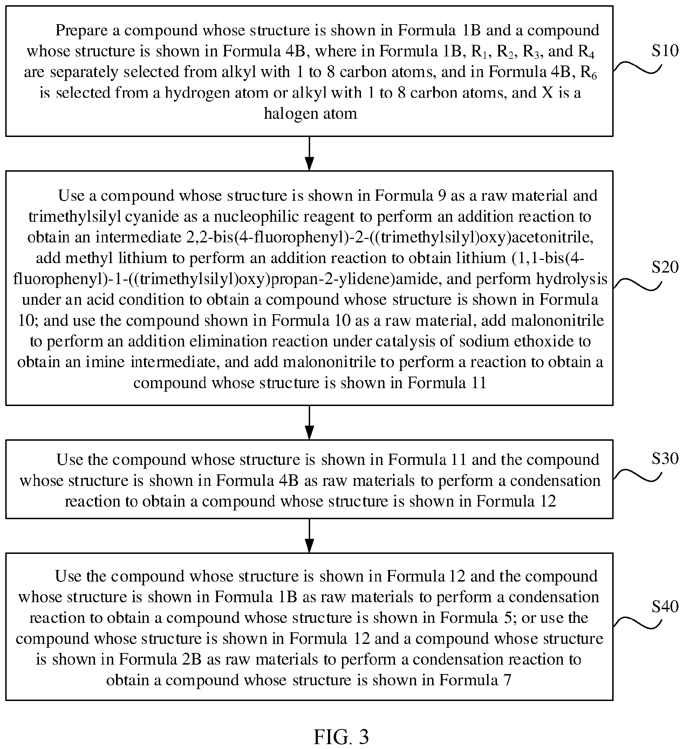

Specifically, with reference to FIG. 3, the method for preparing the chromophore provided in this embodiment of this application includes the following steps.

S10: Prepare a compound whose structure is shown in Formula 1B and a compound whose structure is shown in Formula 4B, where in Formula 1B, R₁, R₂, R₃, and R₄ are separately selected from alkyl with 1 to 8 carbon atoms, and in Formula 4B, R₆ is selected from a hydrogen atom or alkyl with 1 to 8 carbon atoms, and X is a halogen atom.

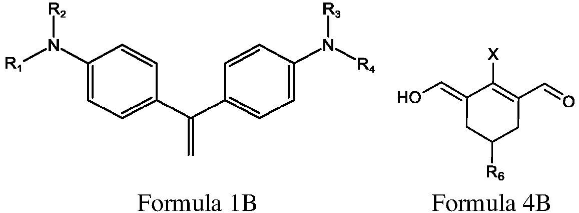

In this step, the compound whose structure is shown in Formula 1B is used as a donor for preparing the chromophore, and is combined with a conjugated bridge to form the foregoing structure shown in Formula 1A. The compound whose structure is shown in Formula 4B is used as a conjugated bridge for preparing the chromophore, and is combined with a donor and an acceptor to form the foregoing structure shown in Formula 4A. In this embodiment of this application, a preparation sequence of the compound shown in Formula 1B and the compound shown in Formula 4B is not limited.

In this embodiment, for selection of R₁, R₂, R₃, and R₄ in the compound shown in Formula 1B, and an example compound of the compound shown in Formula 1B, and for selection of R₆ and X in the compound shown in Formula 4B, and an example compound of the compound shown in Formula 4B, reference may be made to the foregoing descriptions. For brevity, details are not described herein again.

In a possible implementation, the compound whose structure is shown in Formula 1B is prepared by using the following method: making a compound shown in Formula 14 react with methyl lithium to obtain the compound whose structure is shown in Formula 1B. The reaction is shown in the following formula:

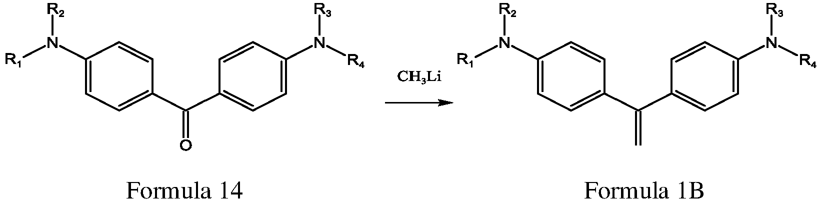

With the use of this method, the Michler's base derivative shown in Formula 1B can be prepared in one step, and a yield can reach 95%.

In a possible implementation, the compound whose structure is shown in Formula 4B is prepared by using the following method: making a compound shown in Formula 15 react with phosphoryl chloride or phosphoryl bromide to obtain the compound whose structure is shown in Formula 4B. The reaction is shown in the following formula:

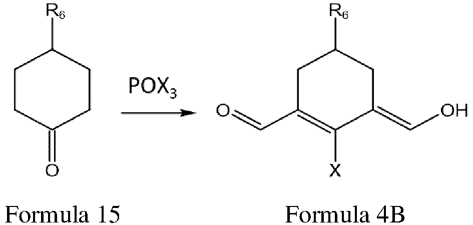

In Formula 4B and POX₃, X is a chlorine atom or a bromine atom.

S20: Use a compound whose structure is shown in Formula 9 as a raw material and trimethylsilyl cyanide as a nucleophilic reagent to perform an addition reaction to obtain an intermediate 2,2-bis(4-fluorophenyl)-2-((trimethylsilyl)oxy)acetonitrile, add methyl lithium to perform an addition reaction to obtain lithium (1,1-bis(4-fluorophenyl)-1-((trimethylsilyl)oxy)propan-2-ylidene)amide, and perform hydrolysis under an acid condition to obtain a compound whose structure is shown in Formula 10; and use the compound shown in Formula 10 as a raw material, add malononitrile to perform an addition elimination reaction under catalysis of sodium ethoxide to obtain an imine intermediate, and add malononitrile to perform a reaction to obtain a compound whose structure is shown in Formula 11.

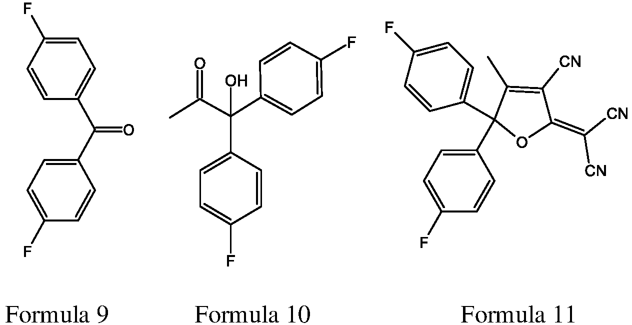

In this step, two consecutive multiple-step one-pot reactions are used to synthesize the acceptor FP-TCF (Formula 11). In some embodiments, reaction steps for the compound shown in Formula 11 are as follows:

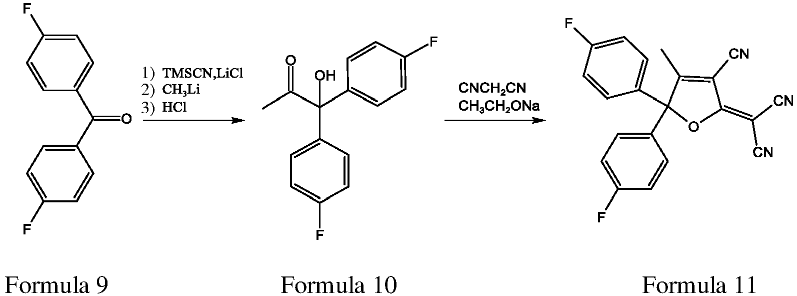

For example, the compound whose structure is shown in Formula 9 is used as a raw material, and under an ice bath condition, lithium chloride and tetrahydrofuran are added, trimethylsilyl cyanide is added dropwise, and a reaction lasts for approximately 3 hours at 50°C, to form an intermediate 2,2-bis(4-fluorophenyl)-2-((trimethylsilyl)oxy)acetonitrile. Ice bath is used again, methyl lithium is added, a room temperature is maintained to make a reaction last for 3 hours, to obtain lithium (1,1-bis(4-fluorophenyl)-I-((trimethylsilyl)oxy)propan-2-ylidene)amide. Then, a 50% hydrochloric acid solution is added dropwise, and a reflux reaction is performed overnight, to hydrolyze lithium (1,1-bis(4-fluorophenyl)-1-((trimethylsilyl)oxy)propan-2-ylidene)amide, and purification is performed by using column chromatography, to obtain the compound whose structure is shown in Formula 10. The compound shown in Formula 10 is used as a raw material, and one equivalent of malononitrile and carbonyl are added to perform an addition elimination reaction under catalysis of sodium ethoxide to obtain an imine intermediate. Then, the intermediate and one equivalent of malononitrile react to remove ammonia molecules, and purification is performed by using column chromatography, to obtain the compound whose structure is shown in Formula 11. For example, time for a reflux reaction with malononitrile and sodium ethoxide added is one day. In this method, a total yield of the acceptor FP-TCF synthesized by using two consecutive one-pot methods is approximately 50%, which is far higher than that of an acceptor CF₃-TCF.

S30: Use the compound whose structure is shown in Formula 11 and the compound whose structure is shown in Formula 4B as raw materials to perform a condensation reaction to obtain a compound whose structure is shown in Formula 12.

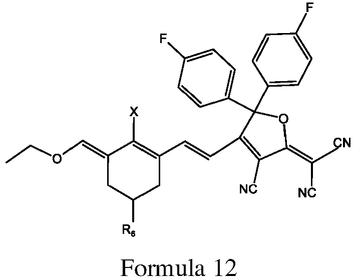

In this step, a reaction formula for a condensation reaction between the compound shown in Formula 11 and the compound whose structure is shown in Formula 4B to obtain the compound shown in Formula 12 is as follows, and a yield of this step is approximately 87%.

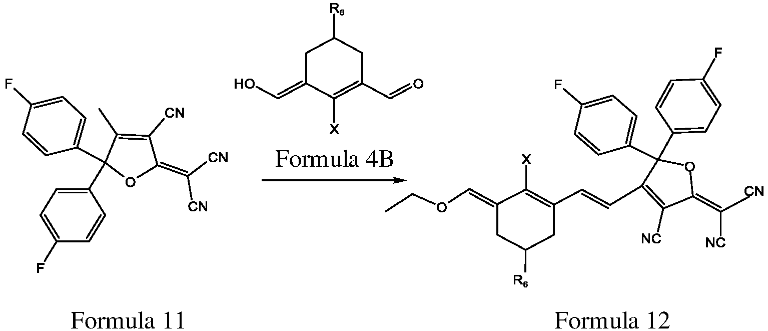

S40: Use the compound whose structure is shown in Formula 12 and the compound whose structure is shown in Formula 1B as raw materials to perform a condensation reaction to obtain a compound whose structure is shown in Formula 5; or use the compound whose structure is shown in Formula 12 and a compound whose structure is shown in Formula 2B as raw materials to perform a condensation reaction to obtain a compound whose structure is shown in Formula 7.

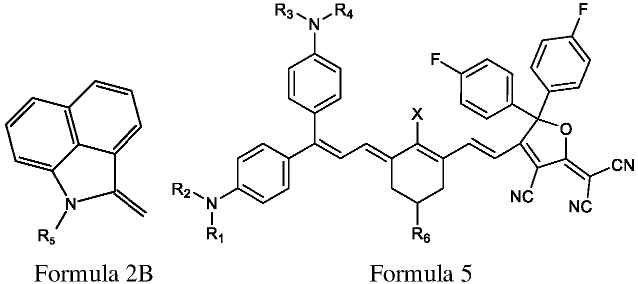

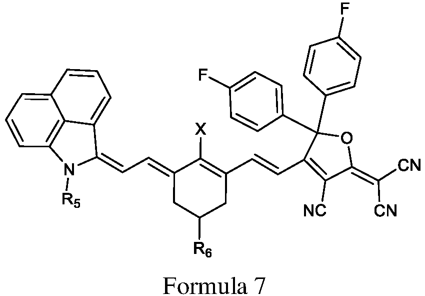

This step includes two methods for preparing the structure shown in Formula 5 and the structure shown in Formula 7 respectively, and a yield of this step is approximately 78%.

In a possible implementation, the compound whose structure is shown in Formula 12 and the compound whose structure is shown in Formula 1B are used as raw materials to perform a condensation reaction to obtain the compound whose structure is shown in Formula 5. A reaction formula of this step is as follows:

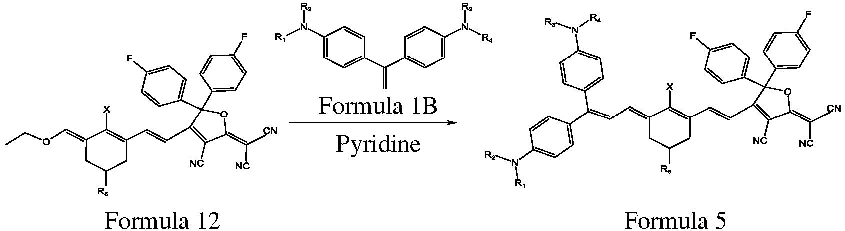

In another possible implementation, the compound whose structure is shown in Formula 12 and the compound whose structure is shown in Formula 2B are used as raw materials to perform a condensation reaction to obtain the compound whose structure is shown in Formula 7. A reaction formula of this step is as follows:

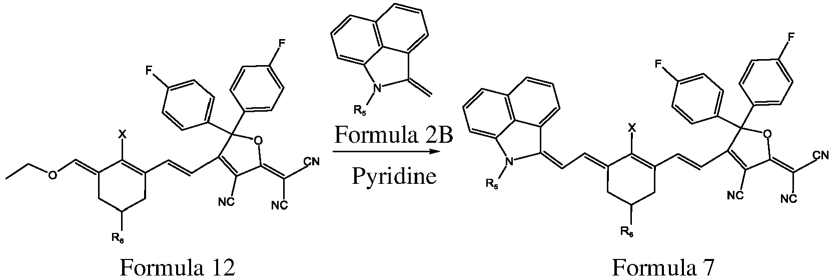

In a possible implementation, nucleophilic substitution is performed on the obtained compound whose structure is shown in Formula 5 or Formula 7, to prepare a chromophore including a 4,5-diphenyl-oxazol-2-ylsulfanyl group. A yield of this step is approximately 68%.

In an implementation, when an obtained compound is the compound shown in Formula 5, the method further includes: using the compound whose structure is shown in Formula 5 and a compound whose structure is shown in Formula 13 as raw materials to perform a nucleophilic substitution reaction to obtain a compound whose structure is shown in Formula 6.

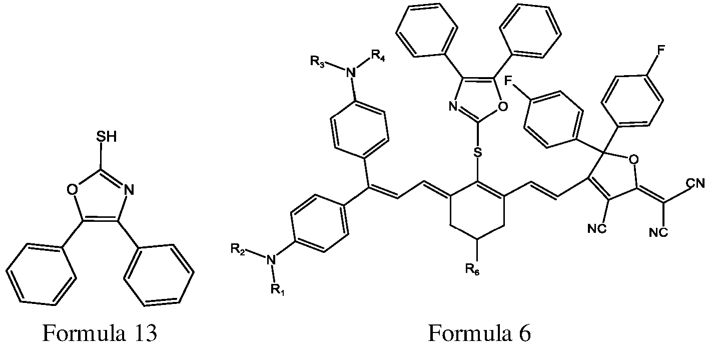

The compound whose structure is shown in Formula 5 and the compound whose structure is shown in Formula 13 perform a nucleophilic substitution reaction to obtain the compound whose structure is shown in Formula 6, and the reaction is as follows:

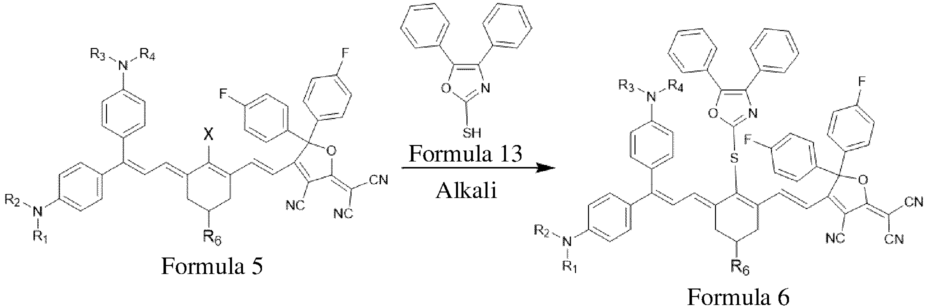

In this implementation, a halogen atom of the structure shown in Formula 5 is replaced by a 4,5-diphenyloxazol-2-ylsulfanyl group to obtain the structure shown in Formula 6. 4,5-diphenyl-oxazol-2-ylsulfanyl has large steric hindrance, and can effectively reduce aggregation of chromophores and reduce π-π stacking of chromophore molecules after being connected to a conjugated group (a part other than 4,5-diphenyl-oxazol-2-ylsulfanyl in the conjugated bridge) that connects the acceptor and the donor. In addition, 4,5-diphenyl-oxazol-2-ylsulfanyl affects weak hydrogen bond interaction between molecules (for example, C-H ... N or C-H ... CI), and increases a polar order parameter, so that the chromophore has a better orientation. Specifically, after the halogen atom is replaced by 4,5-diphenyl-oxazol-2-ylsulfanyl, bond length alternation (BLA) on a main chain is changed obviously. For example, according to single crystal data, BLA of the structure shown in Formula 5 is 0.04(9) Å, and BLA of the structure shown in Formula 6 obtained after sulfhydryl substitution is 0.029 Å. This indicates that the chromophore using a first conjugated group including the 4,5-diphenyl-oxazol-2-ylsulfanyl group as the conjugated bridge has a stronger cyanine-like resonance structure, and a finally obtained polymer material has better nonlinear optical properties and a higher electro-optic coefficient.

In another implementation, when an obtained compound is a compound shown in Formula 6, the method further includes: using the compound whose structure is shown in Formula 7 and a compound whose structure is shown in Formula 13 as raw materials to perform a nucleophilic substitution reaction to obtain a compound whose structure is shown in Formula 8.

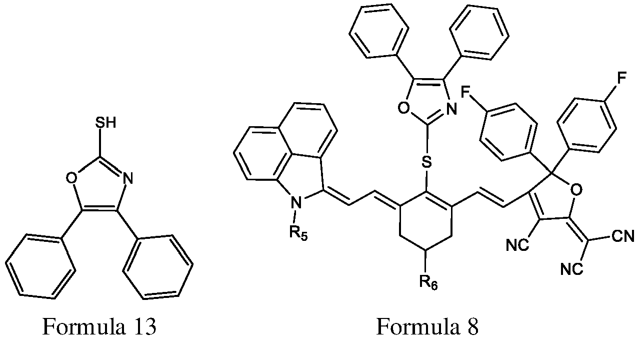

The compound whose structure is shown in Formula 7 and the compound whose structure is shown in Formula 13 perform a nucleophilic substitution reaction to obtain the compound whose structure is shown in Formula 8, and the reaction is as follows:

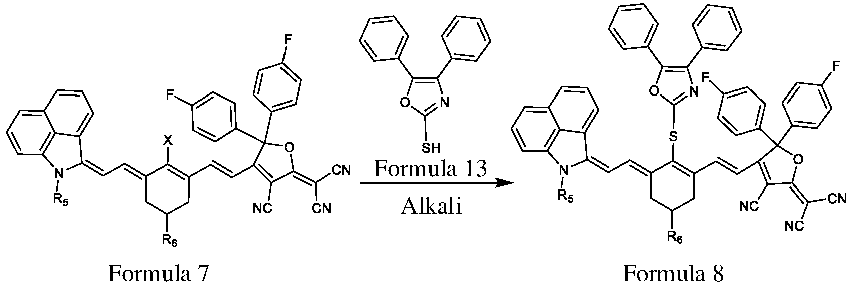

According to the method for preparing the chromophore provided in this embodiment of this application, two consecutive multiple-step one-pot reactions may be used to synthesize the acceptor 2-dicyanomethylidene-3-cyano-4-methyl-5,5-bis(4-fluorophenyl)-2,5-dihydrofuran (FP-TCF), and a total yield is 50%, which is far higher than that of an existing acceptor CF₃-TCF. On this basis, the acceptor is condensed with the conjugated bridge, and then an obtained compound is condensed with a donor to prepare the chromophore. There are few reaction steps and a high yield. In conclusion, the method for preparing the chromophore provided in this application has advantages of a simple process, a high yield, and low costs.

The chromophore prepared according to embodiments of this application has an ultrahigh β value (higher than those of two push-pull tetraene chromophores with best properties in the industry that are based on an acceptor CF₃-TCF and that are difficult to synthesize), has good chemical and thermal stability, and is used to synthesize an organic electro-optic material with a high electro-optic coefficient, high stability, and low costs, so as to implement an electro-optic terminal device with high bandwidth, high efficiency, and low costs that is based on the organic electro-optic material.

The following provides description with reference to specific embodiments.

### Embodiment 1

A chromophore has a heptamethine chromophore molecular structure based on a bis(4-fluorophenyl) (FP) substituted TCF (2-dicyanomethylidene-3-cyano-4-methyl-5,5-bis(4-fluorophenyl)-2,5-dihydrofuran) acceptor (namely, an acceptor FP-TCF), and the molecular structure is as follows:

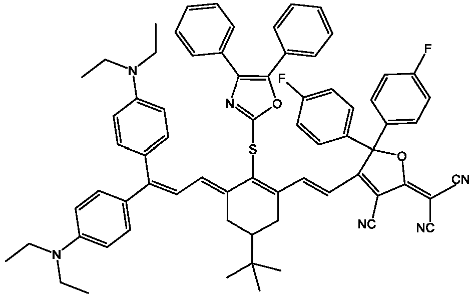

A method for preparing the chromophore is as follows: First, a donor, a conjugated bridge, and an acceptor are synthesized separately, and then assembled. Next, the acceptor is condensed with the conjugated bridge, and then an obtained compound is condensed with the donor. Finally, the chromophore is modified, and a side chain is added. A reaction procedure of steps during preparation is as follows:

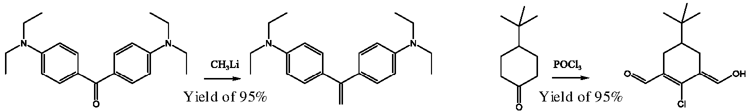

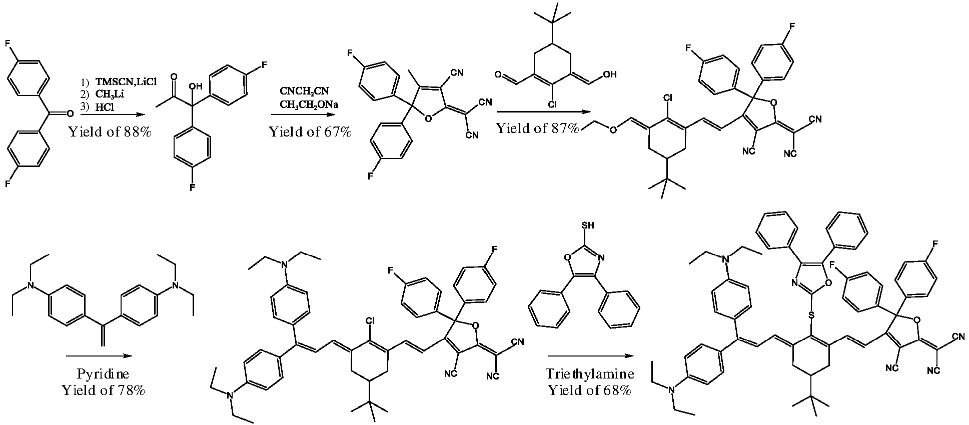

An existing chromophore based on push-pull tetraene requires 11 steps of reaction, a total yield is less than 10%, and a material that is prone to spontaneous combustion is produced in a synthesis procedure. In contrast, there are seven synthesis steps in total for synthesizing the chromophore according to the foregoing reaction procedure, a total yield reaches 25%, and a material that is prone to spontaneous combustion, for example, tert-butyl lithium, is not used. Two consecutive multiple-step one-pot reactions may be used to synthesize the acceptor FP-TCF, and a total yield is 50%, which is far higher than that of an existing acceptor CF₃-TCF. Therefore, a preparation process of the chromophore provided in this embodiment of this application has advantages of a high yield and low costs.

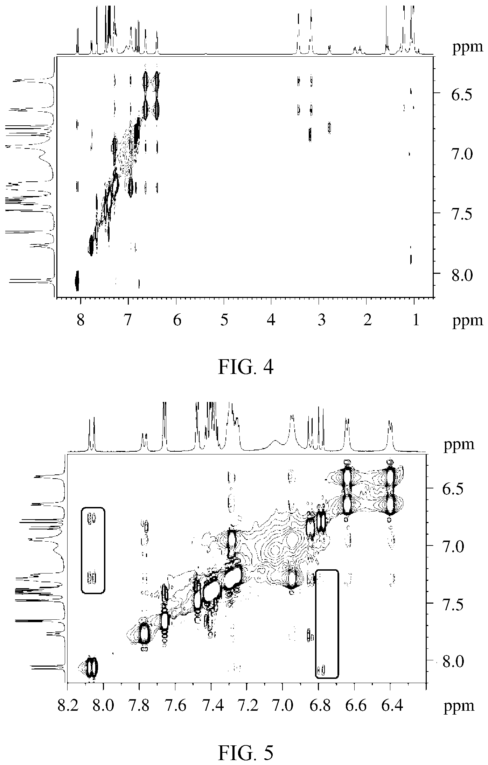

Spectrum measurement is performed on the chromophore prepared in Embodiment 1. An overall spectrum and a partial enlarged view of a two-dimensional ¹H-¹H ROESY (rotating frame overhauser enhancement spectroscopy) spectrum (a rotating frame NOE spectrum) are shown in FIG. 4 and FIG. 5 respectively. During the measurement, the chromophore is dissolved in CDCl₃.

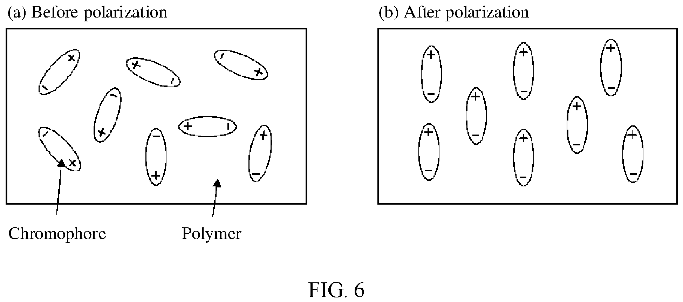

The chromophore provided in Embodiment 1 is doped or chemically bonded to a polymer, to form an organic electro-optic material. Polarization is performed on the organic electro-optic material, to provide photoelectric effect for the organic electro-optic material. As shown in FIG. 6, (a) is a state of the organic electro-optic material before polarization, and (b) is a state of the organic electro-optic material after polarization. It can be learned from the figure that, before the polarization, orientations of chromophore molecules are random. After the polarization, the chromophore molecules present specific orientations on a macroscopic scale, so that the entire organic electro-optic material can have electro-optic effect.

A property test is performed on the chromophore provided in Embodiment 1, including the following indicator tests.
(1) A property test is performed on the chromophore (expressed as M1-FP-ON below) provided in Embodiment 1 and two push-pull tetraene chromophores (expressed as AJLZ53 and AJY-SBu below) based on CF₃-TCF. A test method is specifically as follows: The chromophore and a polymer are mixed at a specific mass ratio, an organic solvent is added for dissolution and even mixture, a film is spin-coated on ITO conductive glass, and after vacuum drying, metal plated is used as a positive electrode and ITO as a negative electrode to prepare a device. An electric field of 100 V/µm is applied, and a temperature is controlled, by using a program, to be raised. When the temperature is around Tg of the polymer, a current is increased sharply. When the current is increased slowly, the temperature is lowered with a voltage maintained, and polarization ends. A prism coupler is used to measure refractive indexes of a thin film under different electric fields, and an electro-optic coefficient and other parameters are calculated based on the refractive indexes. Measurement results of electro-optic coefficients and β values of the chromophore (expressed as M1-FP-ON below) provided in Embodiment 1 and the two push-pull tetraene chromophores (expressed as AJLZ53 and AJY-SBu below) based on CF₃-TCF are shown in Table 1. It should be understood that during comparison, the chromophore is mixed with a polymer poly(styrene-co-methyl methacrylate) (P(S-co-MMA)), and polarization is completed under same conditions. In Table 1, N is a quantity concentration, λₘₐₓ is a maximum absorption wavelength of the thin film, n_{TE} and n_{TM} are birefringence of the thin film, r₃₃ is an electro-optic coefficient at 1.3 µm and 1.54 µm, Φ is an order parameter, and *β* is a hyperpolarizability. Test results at three different concentrations are provided for M1-FP-ON corresponding to Embodiment 1 of this application, and test results at two different concentrations are provided for AJLZ53.

**Table 1**

| Chromophores | *N* 10²⁰ cm⁻³ | *λₘₐₓ* nm | *n_{TE}*/*n_{TM}* at 1.3 µm | *n_{TE}*/*n_{TM}* at 1.54 µm | *r₃₃*, 1.3 µm pm/V | *r₃₃*, 1.54 µm pm/V | *Φ* | <cos³θ> | *β_{µ}* 1.3 µm 10⁻³⁰ esu |
|---|---|---|---|---|---|---|---|---|---|
| **M1-FP-ON** | 1.30 | 866 | 1.6298/1.6774 | 1.6084/1.6542 | 126.8 | 92.8 | 0.1153 | 0.2634 | 8437 |
| **M1-FP-ON** | 0.96 | 867 | 1.6034/1.6392 | 1.5883/1.6132 | 100.6 | 69.8 | 0.1250 | 0.2738 | 8470 |
| **Ml-FP-ON** | 0.67 | 867 | 1.5796/1.6029 | 1.5688/1.5870 | 70.2 | 51.2 | 0.1082 | 0.2553 | 8714 |
| **AJLZ53** | 0.94 | 840 | 1.5943/1.6355 | 1.5800/1.6132 | 97.2 | 56.9 | 0.1339 | 0.2836 | 8056 |
| **AJLZ53** | 0.60 | 834 | 1.5682/1.5907 | 1.5591/1.5746 | 62.3 | - | 0.1165 | 0.2645 | 8160 |
| **AJY-Sβu** | 1.30 | 797 | 1.6039/1.6462 | 1.5891/1.6217 | 94.1 | 59.0 | 0.1171 | 0.2651 | 5984 |

It can be learned from Table 1 that, for an electro-optic material including the chromophore provided in Embodiment 1 of this application, when a concentration is 1.3×10²⁰ cm⁻³, at a wavelength of 1.3 µm, an electro-optic coefficient of the electro-optic polymer reaches 126.8 pm/V, and a β value reaches 8437×10⁻³⁰ esu. At a same mixing concentration, the chromophore in Embodiment 1 presents a highest β value, higher than those of the push-pull tetraene chromophores (AJLZ53 and AJY-SBu) that have best properties currently and that use the strong electron acceptor CF₃-TCF.

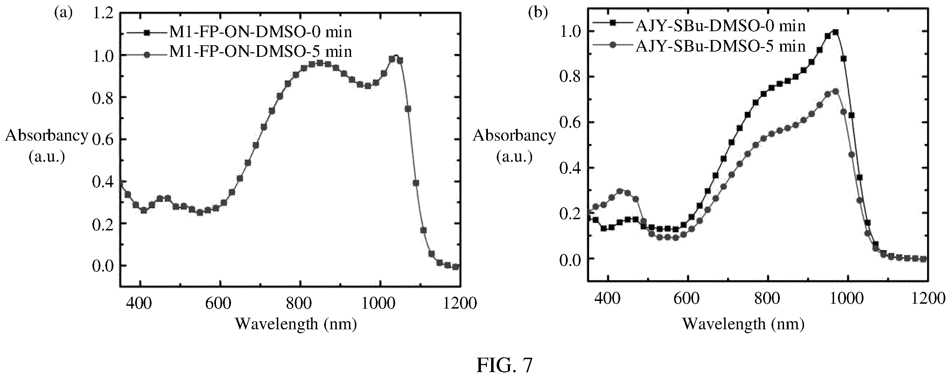

(2) Chemical stability analysis: M1-FP-ON and the tetraene molecule AJY-SBu are separately placed in DMSO (dimethyl sulfoxide, dimethyl sulfoxide) solvents, and UV-vis-NIR (ultraviolet-visible-near infrared) spectra are measured. FIG. 7 shows comparison of chemical stability of the chromophore (Ml-FP-ON, corresponding to (a)) in Embodiment 1 and the push-pull tetraene chromophore (AJY-SBu, corresponding to (b)) based on CF₃-TCF under an alkaline condition. It can be learned from the figure that, after 5 minutes, a maximum absorption peak of AJY-SBu is decreased by 26%, but short wavelength absorption is increased, indicating that AJY-SBu has decomposed, while absorption of M1-FP-ON is almost unchanged, indicating that M1-FP-ON has higher chemical stability than AJY-SBu under the alkaline condition.

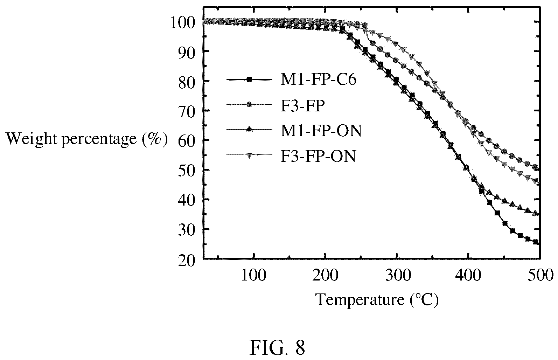

(3) Thermal stability analysis: FIG. 8 shows thermogravimetric analysis (TGA) results of different chromophores based on FP-TCF. It can be learned from the figure that, decomposition temperatures of these chromophores are higher than 200°C, and the decomposition temperature of the chromophore M1-FP-ON in Embodiment 1 is 233°C. It can be learned that the thermal decomposition temperature of the chromophore provided in Embodiment 1 is generally 40°C to 60°C higher than that of a push-pull tetraene chromophore based on CF₃-TCF.

### Embodiment 2

A chromophore has a heptamethine chromophore molecular structure based on a bis(4-fluorophenyl) (FP) substituted TCF acceptor (namely, an acceptor FP-TCF), and is expressed as Ml-FP, and the molecular structure is as follows:

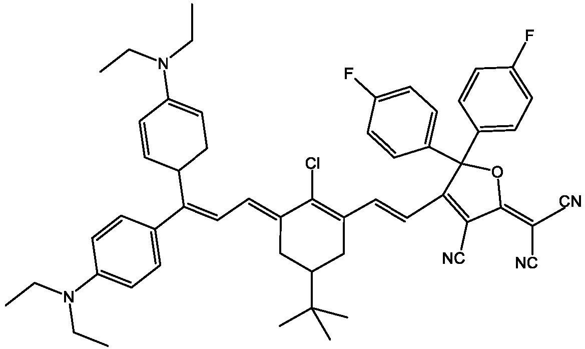

Compared with Embodiment 1 in which a conjugated bridge structure includes a 4,5-diphenyl-oxazol-2-ylsulfanyl group, Embodiment 2 does not have the last step. Therefore, synthesis steps can be reduced, and a synthesis yield can be improved.

### Embodiment 3

A chromophore has a heptamethine chromophore molecular structure based on a bis(4-fluorophenyl) (FP) substituted TCF acceptor (namely, an acceptor FP-TCF), and is expressed as M1-FP-C6, and the molecular structure is as follows:

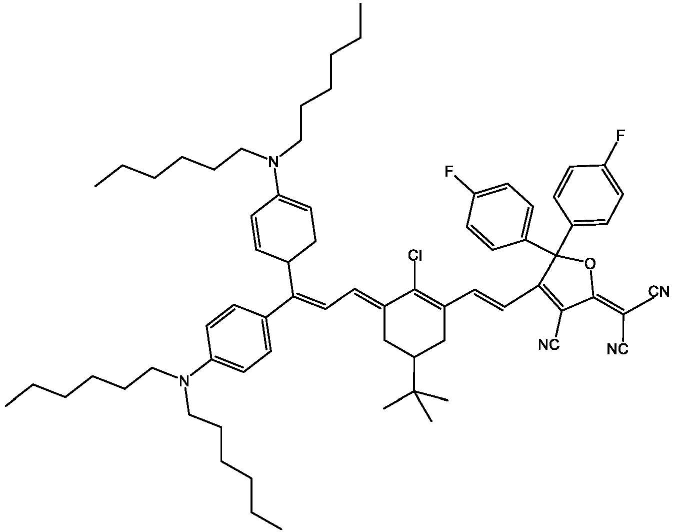

Compared with Embodiment 2, a donor provided in Embodiment 3 includes a plurality of hexyl groups, and can be dissolved in more organic solvents, to improve solubility of the chromophore and facilitate processing and characterization.

### Embodiment 4

A chromophore has a heptamethine chromophore molecular structure based on a bis(4-fluorophenyl) (FP) substituted TCF acceptor (namely, an acceptor FP-TCF), and is expressed as F3-FP-ON, and the molecular structure is as follows:

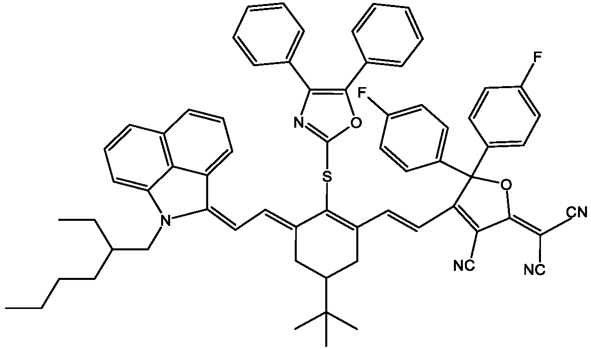

In Embodiment 4, benzo[cd]indoline (benzo[cd]indoline) is used as an electron donor of the chromophore. Compared with Embodiment 1 in which a Michler's base derivative is used as a donor, the chromophore provided in Embodiment 4 can increase a thermal decomposition temperature of the chromophore. As shown in FIG. 8, the decomposition temperature of the chromophore M1-FP-ON is 233°C, and the decomposition temperature of the chromophore F3-FP-ON in Embodiment 4 reaches 281°C.

### Embodiment 5

A chromophore has a heptamethine chromophore molecular structure based on a bis(4-fluorophenyl) (FP) substituted TCF acceptor (namely, an acceptor FP-TCF), and is expressed as F3-FP, and the molecular structure is as follows:

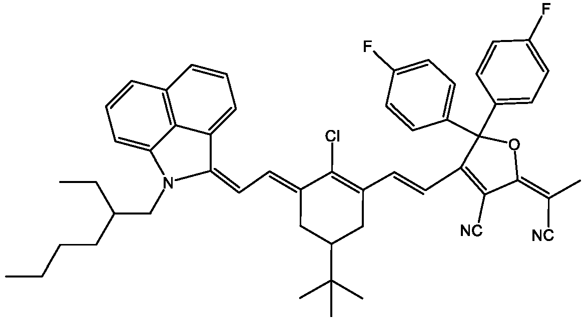

Compared with Embodiment 4, a conjugated bridge structure including a chlorine atom is used in Embodiment 5. Compared with a conjugated bridge structure including a 4,5-diphenyl-oxazol-2-ylsulfanyl group, in this embodiment, one synthesis step can be reduced, and a synthesis yield can be improved.

The foregoing descriptions are merely example embodiments of this application, but are not intended to limit this application. Any modification, equivalent replacement, and improvement made without departing from the spirit and principle of this application shall fall within the protection scope of this application.

## Claims

1. A chromophore, comprising a conjugated bridge, and an acceptor and a donor respectively connected to two ends of the conjugated bridge via a chemical bond, wherein the acceptor is 2-dicyanomethylidene-3-cyano-4-methyl-5,5-bis(4-fluorophenyl)-2,5-dihydrofuran.

2. The chromophore according to claim 1, wherein the donor is a Michler's base derivative donor or a benzo[cd]indoline donor;
a structure of the Michler's base derivative donor is shown in Formula 1A, wherein R₁, R₂, R₃, and R₄ are separately selected from alkyl with 1 to 8 carbon atoms; and 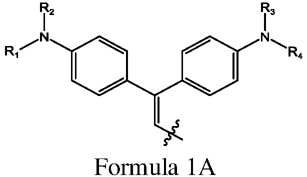
a structure of the benzo[cd]indoline donor is shown in Formula 2A, wherein R₅ is selected from alkyl with 1 to 8 carbon atoms.

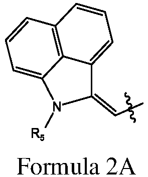

3. The chromophore according to claim 1 or 2, wherein the conjugated bridge is a first conjugated group comprising a 4,5-diphenyl-oxazol-2-ylsulfanyl group or a second conjugated group comprising a halogen atom.

4. The chromophore according to claim 3, wherein a structure of the first conjugated group is shown in Formula 3A: 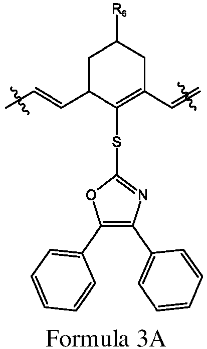
wherein R₆ is selected from a hydrogen atom or alkyl with 1 to 8 carbon atoms; and
a structure of the second conjugated group is shown in Formula 4A: 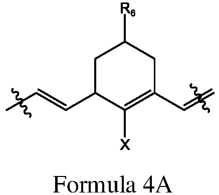
wherein R₆ is selected from a hydrogen atom or alkyl with 1 to 8 carbon atoms, and X is a halogen atom.

5. The chromophore according to claim 1, wherein the chromophore is selected from at least one of structures shown in Formula 5 to Formula 8: 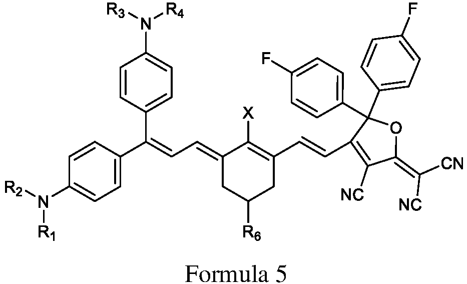 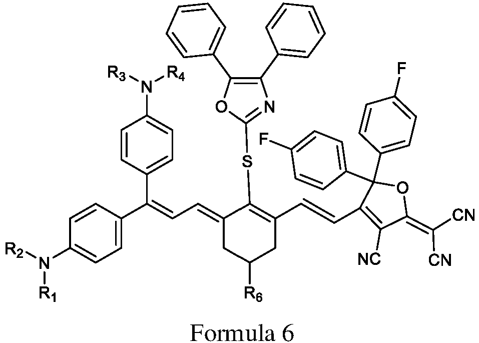 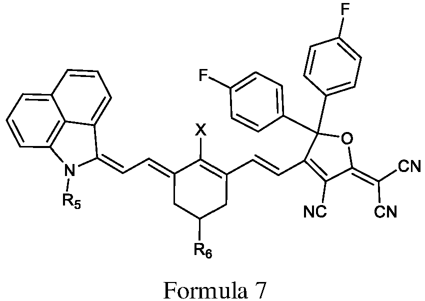 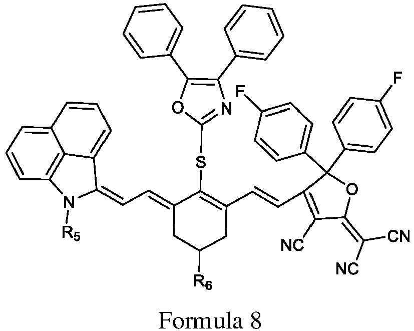 wherein in the formulas, R₁, R₂, R₃, and R₄ are separately selected from alkyl with 1 to 8 carbon atoms, R₆ is selected from a hydrogen atom or alkyl with 1 to 8 carbon atoms, and X is a halogen atom.

6. The chromophore according to claim 5, wherein R₁, R₂, R₃ and R₄ are the same and are selected from ethyl, n-propyl, n-butyl, n-pentyl, or n-hexyl, and R₆ is selected from an H atom, ethyl, isopropyl, or tert-butyl.

7. The chromophore according to any one of claims 1 to 6, wherein the chromophore is selected from one of the following structures:

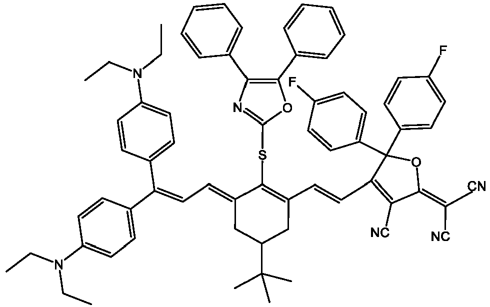

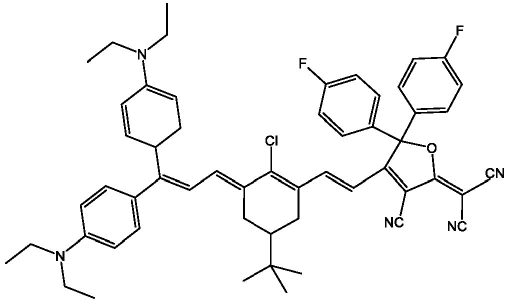

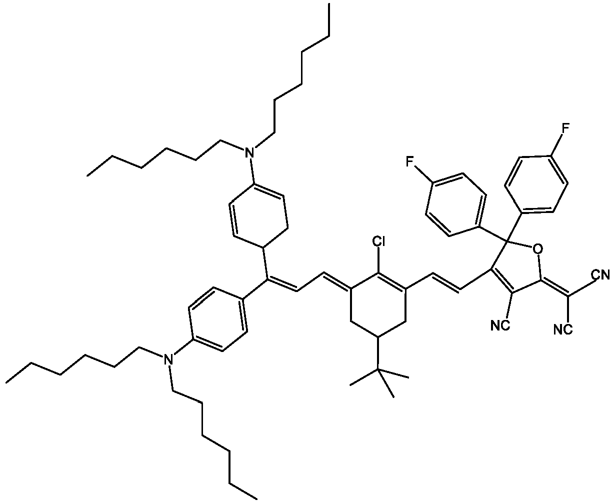

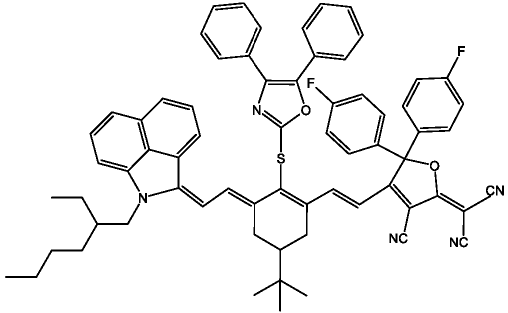

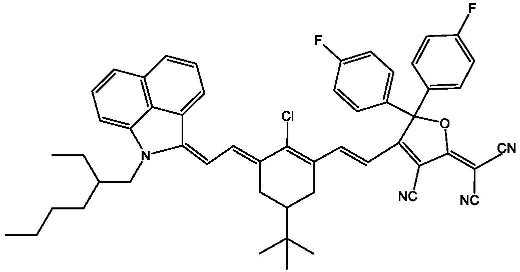

8. A method for preparing a chromophore, comprising the following steps:
preparing a compound whose structure is shown in Formula 1B and a compound whose structure is shown in Formula 4B, wherein in Formula 1B, R₁, R₂, R₃, and R₄ are separately selected from alkyl with 1 to 8 carbon atoms, and in Formula 4B, R₆ is selected from a hydrogen atom or alkyl with 1 to 8 carbon atoms, and X is a halogen atom; 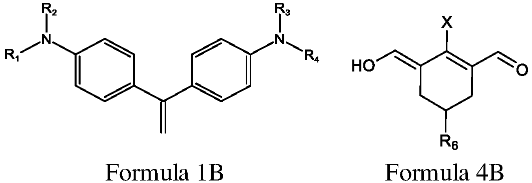
using a compound whose structure is shown in Formula 9 as a raw material and trimethylsilyl cyanide as a nucleophilic reagent to perform an addition reaction to obtain an intermediate 2,2-bis(4-fluorophenyl)-2-((trimethylsilyl)oxy)acetonitrile, adding methyl lithium to perform an addition reaction to obtain lithium (1,1-bis(4-fluorophenyl)-1-((trimethylsilyl)oxy)propan-2-ylidene)amide, and performing hydrolysis under an acid condition to obtain a compound whose structure is shown in Formula 10; and using the compound shown in Formula 10 as a raw material, adding malononitrile to perform an addition elimination reaction under catalysis of sodium ethoxide to obtain an imine intermediate, and adding malononitrile to perform a reaction to obtain a compound whose structure is shown in Formula 11; 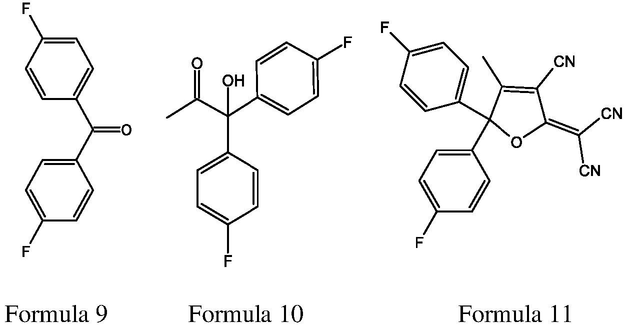
using the compound whose structure is shown in Formula 11 and the compound whose structure is shown in Formula 4B as raw materials to perform a condensation reaction to obtain a compound whose structure is shown in Formula 12; and 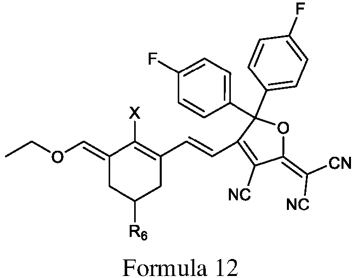
using the compound whose structure is shown in Formula 12 and the compound whose structure is shown in Formula 1B as raw materials to perform a condensation reaction to obtain a compound whose structure is shown in Formula 5; or using the compound whose structure is shown in Formula 12 and a compound whose structure is shown in Formula 2B as raw materials to perform a condensation reaction to obtain a compound whose structure is shown in Formula 7.

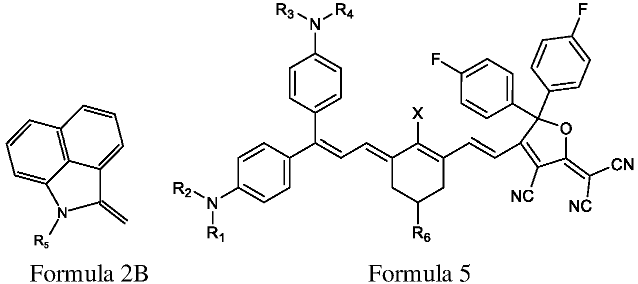

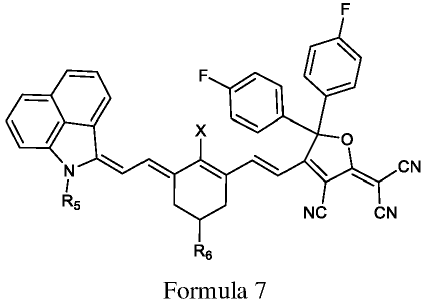

9. The method for preparing the chromophore according to claim 8, wherein when an obtained compound is the compound shown in Formula 5, the method further comprises: using the compound whose structure is shown in Formula 5 and a compound whose structure is shown in Formula 13 as raw materials to perform a nucleophilic substitution reaction to obtain a compound whose structure is shown in Formula 6.

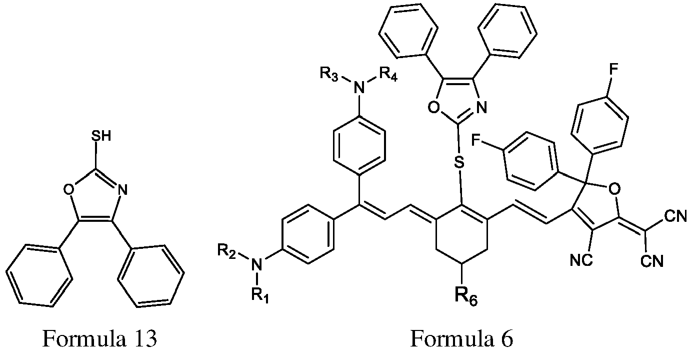

10. The method for preparing the chromophore according to claim 8, wherein when an obtained compound is a compound shown in Formula 6, the method further comprises: using the compound whose structure is shown in Formula 7 and a compound whose structure is shown in Formula 13 as raw materials to perform a nucleophilic substitution reaction to obtain a compound whose structure is shown in Formula 8.

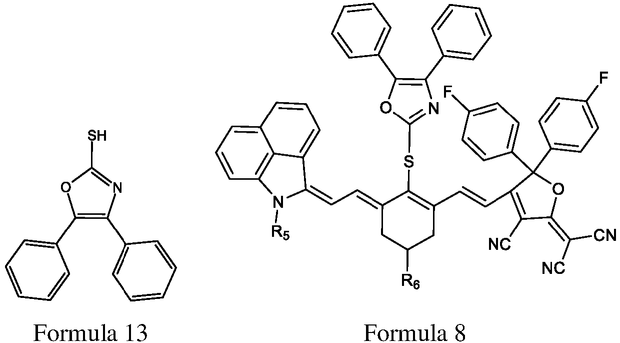

11. The method for preparing the chromophore according to any one of claims 8 to 10, wherein the compound whose structure is shown in Formula 1B is prepared by using the following method: making a compound shown in Formula 14 react with methyl lithium to obtain the compound whose structure is shown in Formula 1B, wherein the reaction is shown in the following formula:

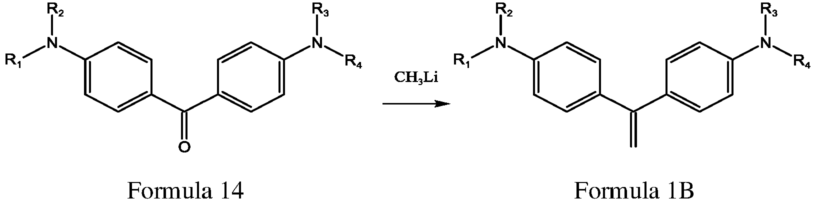

12. The method for preparing the chromophore according to any one of claims 8 to 10, wherein the compound whose structure is shown in Formula 4B is prepared by using the following method: making a compound shown in Formula 15 react with phosphoryl chloride or phosphoryl bromide to obtain the compound whose structure is shown in Formula 4B, wherein the reaction is shown in the following formula: 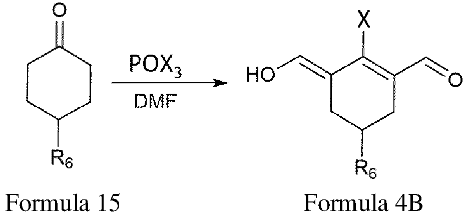 wherein in Formula 4B and POX₃, X is a chlorine atom or a bromine atom.

13. An organic electro-optic material, comprising a polymer and a chromophore, wherein the chromophore is doped to the polymer, or the chromophore is chemically bonded to the polymer, and the chromophore is the chromophore according to any one of claims 1 to 7 or the chromophore obtained by using the method according to any one of claims 8 to 12.

14. An electro-optic terminal device, wherein at least one component of the electro-optic terminal device comprises the organic electro-optic material according to claim 13.

15. The electro-optic terminal device according to claim 14, wherein the electro-optic terminal device comprises an electro-optic modulator, an electric field sensor, or a wireless signal receiver.
